(19) **Europäisches Patentamt**

**European Patent Office**

**Office européen des brevets**

(11) **EP 0 467 952 B1**

(12) **EUROPEAN PATENT SPECIFICATION**

(45) Date of publication and mention
of the grant of the patent:
**04.09.1996 Bulletin 1996/36**

(51) Int Cl.$^6$: **G01N 1/10**, G01N 1/14,
G01N 1/18, G01N 15/05,
G01N 15/12, G01N 15/14,
G01N 27/27

(21) Application number: **90906509.6**

(22) Date of filing: **06.04.1990**

(86) International application number:
**PCT/US90/01880**

(87) International publication number:
**WO 90/13013 (01.11.1990 Gazette 1990/25)**

(54) **METHOD FOR SCREENING CELLS OR FORMED BODIES WITH POPULATIONS EXPRESSING SELECTED CHARACTERISTICS UTILIZING ONE SENSING PARAMETER**

VERFAHREN ZUM SICHTEN VON ZELLEN ODER GEFORMTEN KÖRPERN MIT AUSGEWÄHLTEN EIGENSCHAFTEN UNTER BENUTZUNG EINES NACHWEISPARAMETERS

PROCEDE SERVANT A TRIER DES CELLULES OU DES CORPS FORMES AYANT DES POPULATIONS QUI EXPRIMENT DES CARACTERISTIQUES SELECTIONNEES EN UTILISANT UN PARAMETRE DE SONDAGE

(84) Designated Contracting States:
**AT BE CH DE DK ES FR GB IT LI LU NL SE**

(30) Priority: **14.04.1989 US 339156**

(43) Date of publication of application:
**29.01.1992 Bulletin 1992/05**

(73) Proprietor: **COULTER CORPORATION**
**Hialeah, FL 33010 (US)**

(72) Inventors:
• **RUSSELL, Thomas**
**Miami, FL 33186 (US)**
• **HAJEK, Constance, Mary**
**Miami, FL 33015 (US)**
• **RODRIGUEZ, Carlos, M.**
**Miami, FL 33165 (US)**
• **COULTER, Wallace, H.**
**Miami Springs, FL 33166 (US)**

(74) Representative: **Perry, Robert Edward et al**
**GILL JENNINGS & EVERY**
**Broadgate House**
**7 Eldon Street**
**London EC2M 7LH (GB)**

(56) References cited:
**EP-A- 0 193 356**      **WO-A-88/07199**
**WO-A-90/07259**       **US-A- 4 219 411**
**US-A- 4 452 773**      **US-A- 4 599 307**
**US-A- 4 747 685**

• **Clinical Chemistry, Vol. 23, No. 8 issued 1977, LEIF, et al. "Development of Instrumentation and Fluorochromes for Automated Multiparameter Analysis of Cells", pages 1492-1498 see pages 1498 in particular.**

## Description

## Technical Field

This invention relates generally to a method for screening cells or formed bodies for the enumeration of populations which express selected characteristics, for research, diagnostic or industrial purposes.

## Backgound Art

Automation of routine complete blood cell (CBC) analysis of human peripheral blood by an automated blood cell counter was successfully achieved by the COULTER COUNTER Model A of Coulter Electronics, Inc. of Hialeah, Florida. The electronic particle sensing system principle of that instrument is disclosed in US-A-2,656,508. The use of optical sensing means or lasers, which can be troublesome and expensive is avoided by particle analyzing instrumentation solely operated on this coulter electronic sensing principle.

This Coulter sensing principle was developed and expanded into more sophisticated instrumentation such as the COULTER COUNTER@ Model S types of instruments which enabled CBC parameters, absolute cell counts, platelet count and morphology, red blood cell (RBC) morphology, interpretation of normal and abnormal blood specimens by specific computer programs.

The Coulter electronic particle sensing principle employs an aperture sensing circuit using a direct current (DC) aperture supply. Such particle sensors are simple in structure, extremely rugged and reliable as attested to by the substantially universal acceptance of the COULTER COUNTER@ automated analyzer in clinical laboratories in the United States and throughout the rest of the World. An improvement in this basic aperture sensing circuit was disclosed in U.S. Patent No.. 3,502,974 issued in 1970 to Wallace Coulter and Walter Hogg. In addition to the standard direct current aperture supply, a high frequency aperture current was applied which enabled the sensing of an additional parameter for classification purposes. The high frequency aperture current produced a signal which is the function of the blood cell's internal conductivity as well as its volume. The signal produced simultaneously by the direct current aperture circuit is a conventional DC amplitude signal which provides an indication primarily of cell volume. The radio frequency amplitude is divided by the direct current pulse amplitude employing a high speed divider circuit to obtain a quotient which is a function of cell volume and internal resistance, conveniently referred to as "opacity". This principle is further described in U.S. patent No. 3,502,973 also issued to Wallace Coulter and Walter Hogg, in 1970. This parameter has applicability in cell classification systems. Either a single or a pair of separate apertures could be utilized for this purpose.

Classification of different populations is accomplished by collating the data of the signal pairs as they are produced; one, a measure of particle volume and the other a measure of cell internal resistivity or opacity. A convenient form of presenting this data is by two-dimensional plots referred to as scatterplots or scattergrams. Such plots are well described in Flow Cytometry and Sorting, page 371; edited by Melamed Melaney, and Medelsohn, 1979, John Wiley & Sons, NY, NY.

FIG. 5A of WO-A-8807199 is one example of a data plot of a sample of normal blood. Each dot represents an individual cell. The height above the baseline represents the relative volume of the cell. The distance of the dot to the right of the vertical baseline represents the relative opacity. A plot of normal white blood cells (WBC) (with the red blood cells removed) shows three clusters of dots representing three distinct populations which are a consequence of their intrinsic differences in size and internal composition. If desired, with suitable circuitry, these populations can be enumerated to obtain the numbers of each. The cells are classified on the basis of these inherent differences.

Initial applications of the Coulter electronic particle sensing principle was to perform red blood cell counts and then, more sophisticated determinations of other red blood cell parameters. By removing red blood cells from whole peripheral blood, analysis of the white blood cell populations could be undertaken so long as the red blood cell removal did not significantly impair properties of the remaining white blood cell populations sought to be measured. Red blood cell lysing reagents were developed for this purpose which, though useful and widely applied, were not entirely satisfactory in all respects for subsequent white blood cell determinations.

Previous methods of flow analysis of leukocytes using DC volume alone or light scatter at various angles have shown three clusters of leukocytes corresponding to lymphocytes, monocytes and granulocytes which included the neutrophil, basophil and eosinophil populations. A rough but useful estimation of eosinophil concentration can be made on some samples. The fifth major population is relatively too small for this approach. The eosinophils also have been observed as a distinct cluster using special fluorescence techniques.

These fluorescent techniques were utilized in flow cytometry instruments such as the EPICS@ flow cytometer available from the Coulter corporation. Such instruments employed the principle of cells moving in a columnar stream bounded by a sheath flow such that cells lined up in single file and passed individually through a laser beam. Light scatter and/or fluorescence signals from the cells were then utilized in classifying cell populations. Staining cells with absorptive or fluorescent dyes made additional cell population classifications possible. The development of instrumentation and fluorochromes for automated multiparameter analysis is further described by R. C. Leif, et al. in Clinical Chemistry, Vol. 23, pp. 1492-98 (1977). These developments expanded the number of simultaneous population classifications of leukocytes to four, namely lymphocytes, monocytes, eosinophils and "granulocytes" (neutrophils and basophils).

A more recent analytical hematology instrument has utilized light scattering techniques together with peroxidase enzyme staining (absorptive dye) of cells to produce a five part leukocyte differential. Moreover, dyes in combination with specific reacting biological molecules, such as monoclonal antibodies, have increased the number of leukocyte classifications possible to include functional subdivisions.

WO-A-8807199 discloses a single automated instrument, and methods of using the same, which combine the application of electronic sensing aperture principles, the specificity of selected biological molecules for identifying and/ or enumerating defined populations of cells or formed bodies and microscopic particle technology. The automated analyzer can be used together with a special lysing reagent and/or antibodies coupled to microscopic microspheres or supports of varying composition.

Selectively attaching microscopic particles makes possible the modification of the parameter(s) responsible for the original location of at least one of the populations. The bulk addition of microscopic particles to selected target populations where this addition affects the measured volume and/or opacity results in shifting the location of the dots representing a population.

Antibodies of known specificity are employed in coating microscopic particles. This coating gives the particle the capacity to selectively attach to certain cells which express the antigen the antibody is specific for. These coated or tagged cells are a combination of particles and cell which behave like a new entity. Their parameters of opacity, volume, or both opacity and volume may be considered to represent the sum of the effects of both the cell and the particles on the signals obtained. If the characteristics of the components are different, the new entity will move to a new position in accordance with the net effect. The new location, in contrast with the former position of the cell alone, should allow a classification of such new entity or group of new entities. If the particles attached to the cells are magnetic, then, of course, according to current practice, the new entities can be captured by the use of a magnet. If mixed rapidly, unexpected results including complete capture of a population without adversely affecting the properties of the cells under study occur.

Only three distinct populations of cells can be readily identified and enumerated from a blood sample by utilizing their inherent and unique properties of DC volume and opacity parameters heretofore stated. Additional steps such as improved lysing systems, must be taken to enable the detection and enumeration of more populations. Of course, these additional populations represent subpopulations of the three basic ones referred to as lymphocytes, monocytes and granulocytes. The steps performed in accordance with the parent application demonstrate how subpopulations of these basic three populations are obtained.

Employing such simple aperture sensing techniques in combination with two or more biological particles, one can produce a unique and new position of the dot cluster representing a given population. This selective movement of populations on the dot plot or scattergram is reproducible and can be used to classify a population separate from the basic three populations.

The original and inherent combination of DC volume and opacity sensing techniques can be modified through the attachment of microscopic particles to selected individual cells. The selectivity is given the particles by the nature or specificity of the biological molecules, antibodies among others, employed as the coating on their surfaces. A population of cells alone, having no particles on their surface, may occupy a dot plot position no different from other populations or subpopulations, and, henceforth, not be distinguishable from one another. The addition of particles having a selective attraction to a specific population of cells which one seeks to identify, enumerate, and study is possible using this approach. The selective addition of a sufficient mass of selective particles to a distinct population of interest results in the shifting of that population's dot plot location as a result of the new and unique combination of mass, volume and opacity.

The separation of specific cell populations is accomplished without materially affecting the properties of remaining cell populations. For example, the removal of erythrocytes or red blood cells (RBC's) from whole blood in accordance with this invention permits the measurement of T4 and/or T8 lymphocytes not otherwise possible with heretofore available chemical RBC lysing reagents. Ratios of the number of T4 versus T8 cells have been used to indicate immune deficiencies consistent with severe viral infections including the AIDS virus among others. The presence of specific receptors on the surface of cells can be used to classify a population into subsets, whose enumeration permits the detection of the onset of disease. For example, in the predominant forms of leukemia there is a sharp rise in peripheral blood lymphocytes. If the subpopulation of lymphocytes which is rapidly proliferating bears the T11 receptor, the patient is at risk of immune abnormalities.

Further, if the subpopulation of T11 positive lymphocytes is T4 receptor bearing, then the patient is classified as that common in Japan. These cells are defined as "overlapping" since the cells include at least two receptors or antigens of interest. overlapping can be a significant parameter in diagnosis and treatment. An example of overlapping populations in a normal whole blood sample is the CD2 and CD8 subset populations. Another example of an abnormal overlapping of populations is found in CLL (chronic lymphocytic leukemia). In the CLL disease state, the CD5 and CD20 subset populations overlap. Moreover, if the T4 receptor subpopulations expanding is 2H4 positive, then the patient will not only demonstrate a tendency of multiple infections but acute leukemia as well for the T11, T4, 2H4 positive cell is the inducer of suppression and functionally inhibits the patient's ability to make antibodies. Therein the patient is subject to multiple infections and must be treated for both leukemia and immune deficiency. K. Takatsuki, et al., GANN monograph on Cancer Research 28:13-22, 1982; C. Morimoto, et al., Coulter Japan Symposium, 1984; C. Morimoto, et al., Immunology 134 (3):1508-1515, 1985; C. Morimoto, et al., New England Journal of Medicine 316(2): 67-71, 1987.

## Summary of the Invention

While WO-A-8807199 discloses a method of determining the percentage of at least one white blood cell (WBC) population or subset population in a whole blood sample, which comprises:

analysing first and second portions of the sample, after removing the red blood cell population without significantly adversely affecting relative qualities and/or quantities of WBC populations and/or subset populations, to determine their respective WBC population or subset population characteristics; and comparing the two analysed characteristics;

wherein the second portion has been treated by substantially depleting at least one WBC population or subset population thereof, by mixing therewith microspheres having bonded thereto a monoclonal antibody specific to the at least one WBC population or subset population; and wherein each analysing step is by Coulter sensing or light-scattering techniques, the method of the present invention utilises a single electronic or light parameter.

The invention can be utilised in diagnosing, monitoring or treating patients. Specifically, the invention can be utilised to eliminate or shift populations, to analyse populations or subpopulations which cannot otherwise easily be identified. The change in parameter can be sensed without regard to the substrate, or lack thereof.

The detection of multiple leukocyte subpopulations, and their relationship to one another in human peripheral blood, are important in medical research and the diagnosis of human diseases. Such data are useful as a screening tool for identifying and classifying diseases such as leukaemia. The present invention can be used to identify abnormal situations, and to provide diagnostically relevant information in areas of study, not limited only to detection of leukocyte populations.

One of the most valuable features of this invention is that it employs the single rugged Coulter sensing principle. Apparatus for use in the invention is thus stable, and does not require the complexity and expense of complex optical systems, but can utilise light-sensing if desired. The circuitry required for the addition of a RF generator and detector is economical, compact and reliable. A single aperture is all that is required but the addition of a second or even a third aperture can enable a greater sample throughput, economically.

## Description of the Invention

The novel method can be used to provide multiple-part or five-part WBC differentials, lymphocyte subsets and overlapping determinations. A whole blood sample or portion thereof can be screened to provide the desired analysis of the WBC populations, by elimination of populations and/or subsets from the sample. If desired, the overlapping of antigens on at least two WBC subset populations is determined by depleting each of the WBC subset populations from separate sample portions, and analysing and comparing the results with those from the original sample.

The invention will now be described by way of example only with reference to the accompanying drawings, in which:

FIG. 1 is a schematic block diagram of one single sensing parameter multipart WBC population subset analyzer for use in the invention;

FIG. 2 is one specific analyzer embodiment corresponding to FIG. 1;

FIGS. 3A-D are scattergrams of one set of results utilizing a prototype analyzer system and a DC sensing parameter similar to that illustrated with respect to FIGS. 1 and 2;

FIGS. 4A-D are scattergrams of the same results of FIGS. 3A-D utilizing an RF sensing parameter;

FIGS. 5A-D are scattergrams of a second set of results utilizing the prototype analyzer system and a DC sensing parameter similar to that illustrated with respect to FIGS. 1 and 2.

FIGS. 6A-D are scattergrams of the same results of FIGS. 5A-D utilizing a RF sensing parameter;

FIGS. 7-10 are scattergrams of results illustrating the use of a single light sensitive parameter;

FIG. 11 is a schematic block diagram of a WBC population subset analysis for enhancing small or obscure populations;

FIGS. 12A-E are scattergrams of one set of results obtained utilizing a prototype analyzer system and an RF sensing parameter similar to that illustrated with respect to FIGS. 1 and 11;

FIGS. 13A-E are scattergrams of another set of results obtained utilizing a DC sensing parameter;

FIGS. 14A-E are scattergrams of the same set of results utilizing an RF sensing parameter;

FIG. 15 is a scattergram of a control experiment, obtained utilizing two sensing parameters;

FIGS. 16A-G and 17A-G are scattergrams of results obtained utilizing various sensing parameters on two respective abnormal blood samples;

FIG. 18 is a schematic block diagram of a WBC subset population analyzing embodiment of the invention for determining overlapping classification of cells; and

FIGS. 19A-D and 20A-D are scattergrams of two sets of results utilizing a prototype analyzer system and a DC sensing parameter similar to that illustrated in FIGS. 1 and 18.

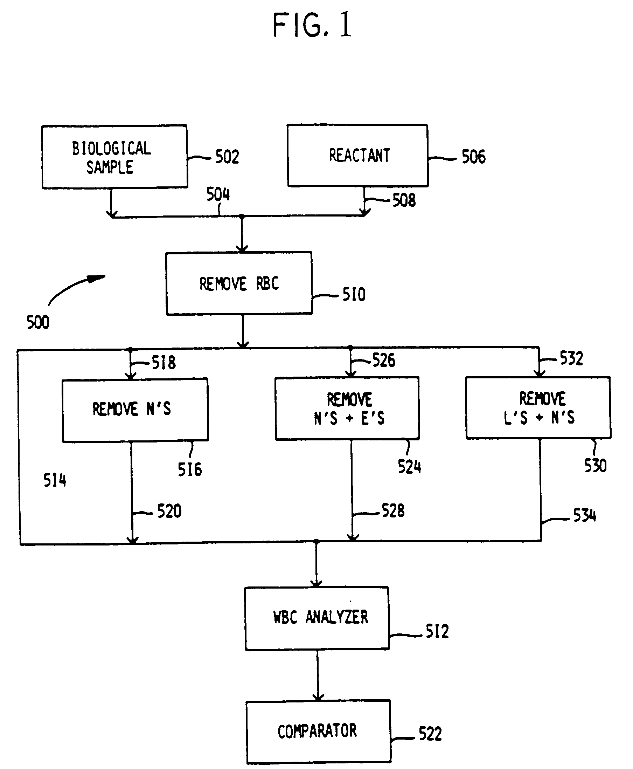

Referring to FIG. 1, apparatus for performing classification of cells such as a multipart differential is designated generally by the reference numeral 500. The instrument or analyzer 500 includes a biological sample 502, which contains at least a first set of viable biological cells (not illustrated), including at least two white blood cell populations, such as in or from a whole blood sample.

The cells of the biological sample 502 are to be involved in a biological reaction in a quantitative and/or qualitative determination or analysis. The biological sample 502 can include a buffer into which the cells are added.

The biological sample 502 is combined via a line 504 with at least one reactant 506 via a line 508. In the analyzer 500, the RBC's are removed from the mixture at an RBC removing station 510. As stated in the first parent application, the RBC's can be removed from the station 510 in a number of ways, such as enumerated with respect to the station 20.

A first portion of the mixture with the RBC's removed, then is fed to a WBC analyzer 512 via a line 514. This obtains a standard or control for the total or whole WBC populations of the biological sample 502. The analyzer 512 can be the same as the analyzer 86 illustrated and described in WO-A-8807199 or can be a light sensing analyzer, such as described in WO-A-8807198. The single sensing parameter can be electronic, such as RF or DC or light, such as median angle light scatter (Scatter) or any other desired light parameter.

A second portion of the mixture is fed to a N removing station 516 via a line 518. The N's are removed by the addition of the appropriate magnetic microspheres with the N specific antibody bound thereto, e.g. as disclosed in WO-A-8804320. A magnet or magnetic field is utilized, as before discussed, to remove the magnetically bound cells from the mixture. The remaining mixture with the N's removed then is fed via a line 520 to the analyzer 512. The analyzed results of this portion of the mixture then can be compared with the analyzed results of the first mixture portion in a comparator 522 to obtain the percentage of N's in the biological sample 502.

A third portion of the mixture is fed to a N and E removing station 524 via a line 526. The N's and E's are removed by the addition of appropriate magnetic microspheres with the N and E specific antibody bound thereto. One such antibody is disclosed in WO-A-8900169. Separate N and E specific antibodies, bound to the same or separate magnetic microspheres, also can be utilized, where appropriate or as developed. The remaining mixture with the N's and E's removed then is fed via a line 528 to the analyzer 512. The analyzed results of this portion of the mixture then is compared to the results of the first and second mixture portions in the comparator 522 to obtain the percentage of E's and M's in the biological sample 502.

A fourth portion of the mixture is fed to a L and N removing station 530 via a line 532. The L's and N's are removed by the addition of the appropriate magnetic microspheres with the L and N specific antibody bound thereto. The N specific antibody can be either of the above referenced N specific antibodies, or other appropriate antibodies. The L specific antibody can be an L specific antibody as developed or a combination of the specific antibodies sold under the nomenclature T11 and 2H4 by Coulter Immunology Division of Coulter Corporation, which together bind all the L's. The remaining mixture with the L's and N's removed then is fed via a line 534 to the analyzer 512. The analyzed results of this portion of the mixture then can be compared to the results of the other mixture portions to obtain the percentage of B's and L's in the biological sample 502.

Thus, the analyzer 500 performs a single classification of cells, such as N's utilizing lines or channels 514 and 518, E's and/or N's and/or M's utilizing lines 514, 518 and 526 and a full five part WBC differential utilizing all four lines. One most important feature of the analyzer 500 is that the mixtures are analyzed utilizing only a single analyzing parameter, such as one electronic parameter or one light parameter. Other combinations can be utilized, but in each case only a single sensed parameter or characteristic is necessary to perform the classification of the invention.

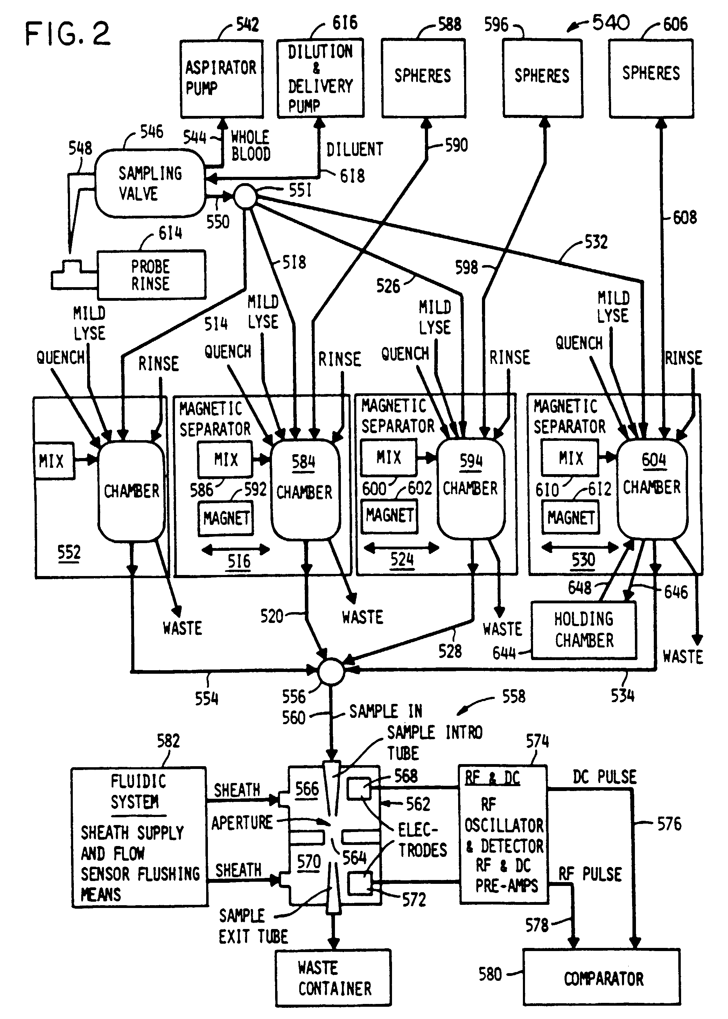

FIG. 2 illustrates a specific analyzing instrument embodiment incorporating the method and apparatus of the analyzer 500, designated generally by the reference numeral 540. The instrument 540 includes an aspirator pumping mechanism 542 which is utilized to draw the biological sample of interest, for example the sample 502, into the instrument 540. The aspirator 542 is coupled via a line 544 to a sampling valve 546, which can be coupled to a sample probe 548. The biological sample 502 then is fed via a line 550 and a multipart valve 551 into the separate channels 514, 518, 526 and 532.

Describing first the channel 514, the sample portion is fed to a chamber 552. The chamber can be a mixing chamber into which the sample and reactant is fed to remove the RBC's. For example, utilizing lyse, the RBC's are lysed in the chamber 552 by adding the appropriate lyse thereto and preferably mixing therewith, then when the reaction is completed, a quench or fix is supplied to the chamber 552.

Specific details of an appropriate mixing apparatus, which can be utilized herein are disclosed in WO-A-8806918. By utilizing the mixer, the reactions are greatly enhanced in speed without significantly damaging the properties of interest of the cells, such as, can occur by raising the reaction temperature. Further, the reactions generally are completed in significantly less than a minute, generally on the order of fifteen seconds or less. This allows a rapid analysis of the automatic high volume analyzer instrument 540.

The quenched reactant mixture with the RBC's removed by the lyse then is fed via a line 554 to a multipart valve 556, or directly to a WBC analyzer 558 via a line 560. The analyzer 558 can be of many physical types in accordance with the counting and sizing techniques described US-A-2,656,508, utilizing light or electronic sensing as embodied in the numerous commercial blood cell counters of Coulter Corporation.

The analyzer 558, in general, includes a flow sensor or sensing chamber 562. The chamber 562 includes a transducer 564 which has an aperture therethrough. The chamber 562 can include a first portion 566 which has a first electrode 568 in contact with the fluid therein. The chamber portion 566 and the electrode 568 can communicate through the sensing aperture with a second chamber portion 570 having a second electrode 572 therein.

The electrodes 568 and 572 are coupled via reactive leads to an RF/DC source and sensing circuit 574. The circuit 574 couples either or both a DC, or low frequency current or signal, and a high frequency signal between the electrodes 568 and 572.

The low frequency signal is utilized to sense the amplitude of a signal pulse caused by a cell passing through the sensing aperture. The high frequency signal can be utilized in the same manner as a single parameter or with the low frequency signal to obtain the electrical opacity of the same cell passing through the sensing aperture.

The measuring of the electrical opacity of cells was described by US-A-3,502,974 and several subsequent patents and publications of Coulter Corporation. One specific circuit which can be utilized herein is disclosed in US-A-4,791,355.

The signals generated by the circuit 574 from the sensed cells are coupled via a DC signal lead 576 and/or an RF signal lead 578 to a comparator 580 (like the comparator 26). The comparator 580 can hold the signal generated from the first portion, i.e., those without the WBC population or population subset subtracted, for a comparison with the results from the subsequent portions described hereinafter.

The analyzer 558 can include a sheath flow to focus the cells in the sensor 562, in the well known manner. The sheath flow can be provided by a fluidic system 582, coupled to the sensor in a known manner. The analyzer 558 has been illustrated with both RF and DC analyzing circuitry for example purposes only. In obtaining the multipart WBC population or subset population characterization of the invention, only a single sensing parameter, electronic or optical, is utilized. In the case of electronic sensing, the parameter can be either DC or RF. Utilizing optical sensing, not illustrated, again only a single parameter such as median angle light scatter is utilized. This one dimension sensing can simplify the instrument 540, as well as decrease the cost thereof.

A second portion of the sample mixture is fed through the valve 551 via the line 518 to the N removing station 516. The station 516 includes a mixer or chamber 584. The mixing chamber 584 has the second mixture portion fed thereto via the line 518. The mixer 584 includes all of the various options above described and, for example, includes a lyse input line for the RBC lyse.

When the lyse is utilized, after mixing as illustrated functionally at 586, then the quench is added via a quench line. At the same time or sequentially, the N's are being removed by the addition of the appropriate magnetic microspheres with the N specific antibody bound thereto from a source of microspheres 588 fed to the chamber 584 via a line 590. A magnet 592 or magnetic field is then utilized to remove the magnetically bound cells on the magnetic microspheres. The mixed and quenched mixture than is fed via the line 520 through the valve 556 and the line 560 to the WBC analyzer 558 to be analyzed as before described. The analyzed mixture with the N's removed then is compared in the comparator 580 to determine the percentage of N's in the sample 502.

A third mixture portion of the sample 502 is fed via the line 550 and the valve 551 via the line 526 to the N and E removing station 524. The station 524 includes a mixing chamber 594. In the third portion, the RBC's are removed by the RBC lyse fed into the chamber 594. The lyse is mixed with the sample portion and then a quench is added via a quench line. The N's and E's are removed by magnetic microspheres having the N and E specific antibody or antibodies bound thereto from a microsphere source 596 fed into the chamber 594 via a line 598. The microspheres are mixed, functionally at 600, and then the bound N and E microspheres are magnetically removed, functionally at 602. The N and E removed mixture than is fed via the line 528 to the valve 556 and via the line 560 to the analyzer 558 to also obtain the above mentioned results.

The instrument can be utilized with the first two channels 514 and 518 to determine the N percentage or with the first three channels 514, 518 and 526 to obtain the N's and E's percentages and/or the M's percentage, as desired. To obtain further WBC population or subset population characterizations, a fourth portion of the sample mixture 502 is fed via the line 550 and the valve 551 via the line 532 to the L and N removing station 530. Again, the RBC's are removed such as by lysing in a chamber 604, and the L's and N's are removed by binding the L's and N's to magnetic microspheres having the L and N specific antibody or antibodies bound thereto from a source 606 fed into the mixing chamber 604 via a line 608. The microspheres are mixed, functionally at 610, and then the magnetically bound L and N microspheres are magnetically removed, functionally at 612.

The L and N removed mixture then is fed via the line 534 to the valve 556 and via the line 560 to the analyzer 558 to obtain the above-mentioned results. Utilizing combinations of the results from the other channels, the percentage of L's and/or B's then can be obtained such as to perform a full five-part WBC differential to obtain the percentage of N's, E's, M's, L's and B's. The mixers include appropriate rinse lines and waste lines and the instrument 540 can include a probe rinse 614 to cleanse the instrument 540 prior to aspirating the next sample or sample portion for analyzing. Further, the sample 502 can be diluted from a source 616 via a line 618 if desired.

Only one specific hardware embodiment incorporating the method and apparatus of the analyzer 500 has been illustrated, but like the embodiments in WO-A-8807199, the analyzing instrument 540 can be implemented in numerous configurations. For example, the analyzer 540 could include a single channel, such as the channel 518 and the portions each can be run sequentially through the station 516 with the appropriate WBC population or populations removed from each portion, as previously described with respect to the separate channels.

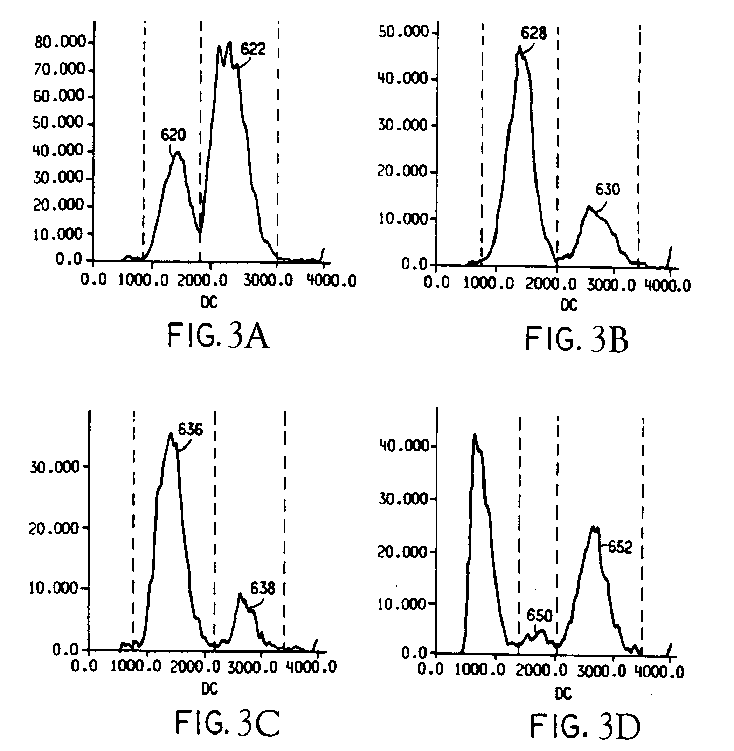

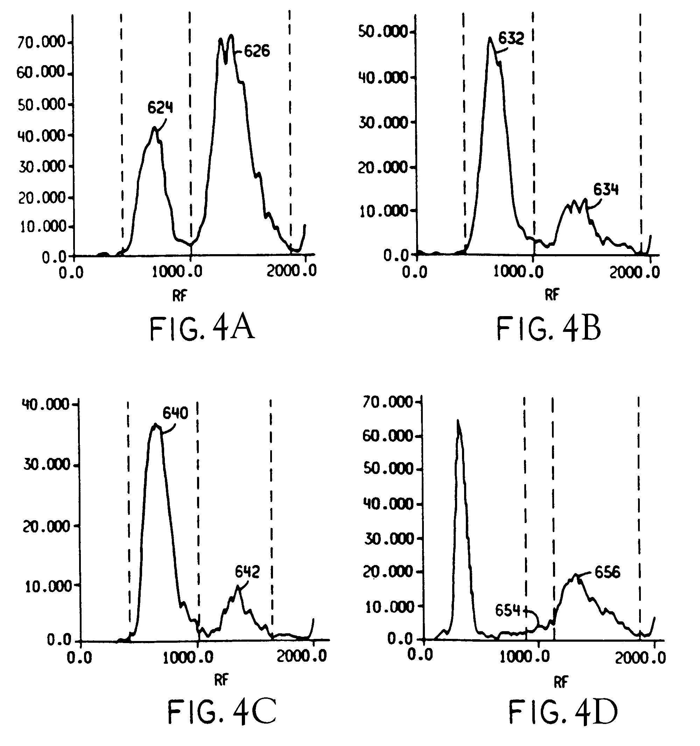

Referring now to FIGS. 3A-D and FIGS. 4A-D, two sets of one - dimensional scattergram multipart characterization results are illustrated, obtained from a whole blood sample, utilizing a prototype analyzing method similar to the analyzer instrument 540. The biological sample in each case was a 28 microliter sample of whole blood, which was combined with 122 microliters of buffer solution for the sample portion utilized in the first channel 514. The sample portion was lysed with 300 microliters of the RBC preferential lyse above referenced in the chamber 552. The sample portion was lysed for 4 seconds and then quenched with 120 microliters of quench before being fed to the analyzer 558 via the line 554, the valve 556 and the line 560. The results of analyzing utilizing a one-dimensional electronic sensing parameter are illustrated in FIGS. 3 and 4. DC was utilized to obtain the data in FIGS. 3A-3D and RF was utilized to obtain the data (for comparison) in FIGS. 4A-4D, utilizing the same sample portion and measured at the same time in the analyzer 558. This results in two clearly identifiable data peaks 620 and 622 in FIG. 3A and peaks 624 and 626 in FIG. 4A. Peaks 620 and 624 are indicative of the percentage of L's and B's in the sample. Peaks 622 and 626 are indicative of the percentage of N's, E's and M's. Clearly, in one dimension, without further manipulation, the individual percentages are masked by the competing cells in the same data peak.

In the second channel 518, a second whole blood sample portion of 28 microliters is combined with 40 microliters of magnetic microspheres with the N specific antibody bound thereto and combined with 82 microliters of buffer solution in the chamber 584. The sample portion was mixed for 60 seconds, lysed and quenched in the same manner as in the channel 514 and then placed in the magnetic field 592 for 30 seconds before the mixture with the N's removed is fed to the analyzer 558 via the line 520, the valve 556 and the line 560. 3 This results in two data peaks 628 and 630 in FIG. 3B and two data peaks 632 and 634 in FIG. 4B. Peaks 628 and 632 remain the same as the peaks 620 and 624 although their percentage of the sample mixture portion now is greater, while the peaks 630 and 634 are indicative of the percentage of the E's and M's with the N's removed. The data peaks 630 and 634 can then be compared with the respective data peaks 622 and 626 to determine the percentage of N's in the whole blood sample.

Next, or in any order including substantially simultaneously, a third 28 microliter sample portion is fed to the third channel 526 and mixed with 20 microliters of magnetic microspheres with the E and N specific antibody or antibodies bound thereto and combined with 102 microliters of buffer solution in the chamber 594. The sample portion was mixed for 30 seconds, lysed and quenched in the same manner as in the channel 514 and then placed in the magnetic field 602 for 30 seconds before the mixture with the N's and E's removed is fed to the analyzer 558 via the line 528, the valve 556 and the line 560. This results again in two data peaks 636 and 638 in FIG. 3C and data peaks 640 and 642 in FIG. 4C. Peaks 636 and 640 again are the same contribution as the peaks 620 and 624, while the data peaks 638 and 642 are indicative of the percentage of the M's in the whole blood sample as compared to the data peaks 622 and 626. The data peaks 638 and 642 then can be compared to the data peaks 630 and 634 also to determine the percentage of E's in the whole blood sample.

A fourth 28 microliter sample portion is fed to the fourth channel 532 and mixed with 70 microliters of magnetic microspheres with a CD2 specific antibody bound thereto and 50 microliters of magnetic microspheres with a CD45R specific antibody bound thereto in the chamber 604. The sample portion was mixed for 2 minutes and then placed in the magnetic field 612 for 30 seconds and then the remaining mixture is removed to a holding chamber 644 via a line 646. The holding chamber 644 currently appears to be a necessary operation, because the specific antibodies utilized as above referenced under the nomenclature T11 and 2H4 and the N specific antibody, appear to interfere with one another when utilized simultaneously. It is not known whether this is due to the specific antibodies or to the nature of the cells themselves. Once the mixture with the T11 and 2H4 bound cells removed is fed into the holding chamber 644, the chamber 604 then is rinsed with the magnetic field removed to remove the magnetic microspheres having the CD2 and CD45R cell clusters bound thereto.

The N bound magnetic microspheres then are isolated in the chamber 604, which can be provided by another source, other than the source 606 (not illustrated). The isolation is accomplished by holding the magnetic microspheres with 40 microliters of the N specific antibody bound thereto in the magnetic field 612 and removing the buffer solution to waste. Alternatively, the mixture can be adjusted so that the buffer solution is utilized as part of the mixture or a concentrated volume of magnetic microspheres can be utilized and the solution need not be utilized. The sample portion then is returned to the chamber 604 from the chamber 644 via a line 648 and mixed with the magnetic microspheres with the N specific antibody bound thereto, lysed and quenched as in the channel 514 and then again placed in the magnetic field 612 for 30 seconds before the mixture with all the L's and N's removed is fed to the analyzer 558 via the line 534, the valve 556 and the line 560. 3 This results in two data peaks 650 and 652 in FIG. 3D and two data peaks 654 and 656 in FIG. 4D. Peaks 650 and 654 represent only the B's, since all the L's have been removed. Since the L's are about 30 percent of a normal whole blood sample, this enhances the B's which originally are about 1 percent of the sample to a very significant data peak (i.e., amount of data). The B's are further enhanced since the N's also were removed from the peaks 652 and 656 and they originally are about 60 percent of the sample. Hence, the B's now represent about 10 percent of the remaining B, M and E cells.

The actual percentage calculations are performed as follows, with the data peak numbers calculated by dividing the data peak (number of cells counted in the peak) by the total data from both peaks (utilizing FIGS. 3A-3D for example purposes):

1) M% - determined from channel 526:

$$[\underline{(Pk1\ (620))}\ \underline{(Pk1\ (636))}]\ xx\ \underline{Pk2\ (638)} = M\%$$

2) M+E% - determined from channel 518:

$$[\underline{(Pk1\ (620))}\ \underline{(Pk1\ (628))}]\ xx\ \underline{Pk2\ (630)} = M+E\%$$

3)

$$E\% = M+E\% - M\%$$

4)

$$N\% = \underline{(Pk2\ (622))} - M+E\%$$

5) B% - determined from channel 532:

$$[\underline{(Pk2\ (630))}\ \underline{(Pk2\ 652))}]\ xx\ \underline{(Pk1\ (650))} = B^1\%$$

$$B^1\%\ xx\ [\underline{(P1(620))}\ \underline{(Pk1\ (628))}] = B\%$$

6)

$$L\% = \underline{(Pk1\ (620))} - B\%.$$

For the data illustrated in FIGS. 3 and 4, the calculations from the RF and DC data are set forth in Table II.

TABLE II

| Channel | DC | | RF | |
|---|---|---|---|---|
| | Pk1 | Pk2 | Pk1 | Pk2 |
| 514 | 28.1 | 71.9 | 30.3 | 69.7 |
| 518 | 74.4 | 25.6 | 74.1 | 25.9 |
| 526 | 83.8 | 16.2 | 83.4 | 16.6 |
| 532 | 10.9 | 90.1 | 10.0 | 90.0 |

A full five part differential in percentage of the N's, L's, M's, E's and B's also was calculated from the DC and RF data peaks and compared to a light sensing instrument for verification purposes, such as described in WO-A-8807198.

TABLE III

| 5 PART DIFFERENTIAL | | |
|---|---|---|
| LIGHT | DC | RF |
| N 61.58 | N 62.2 | N 59.1 |
| L 28.50 | L 26.9 | L 29.1 |
| M 6.27 | M 5.4 | M 6.0 |
| E 3.10 | E 4.3 | E 4.6 |
| B 0.56 | B 1.2 | B 1.2 |

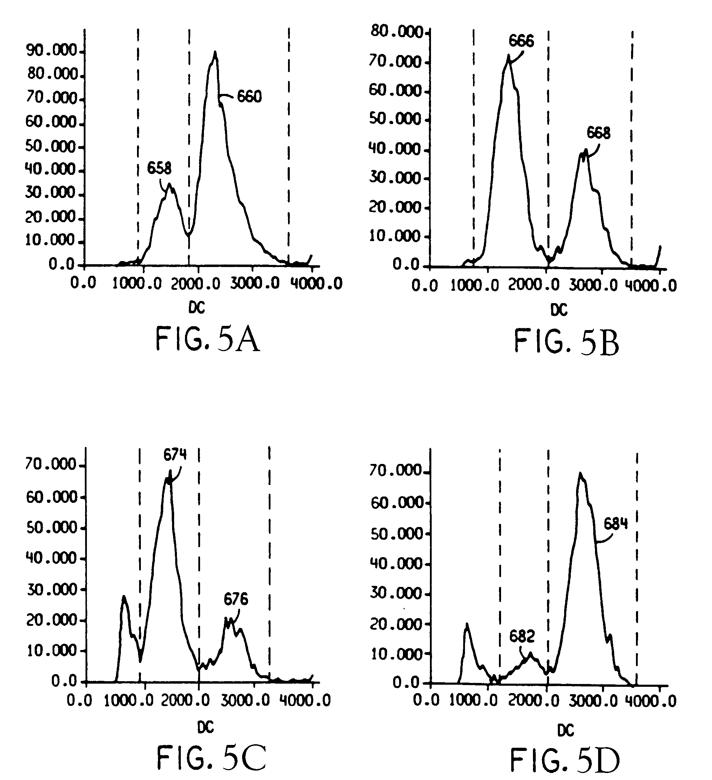

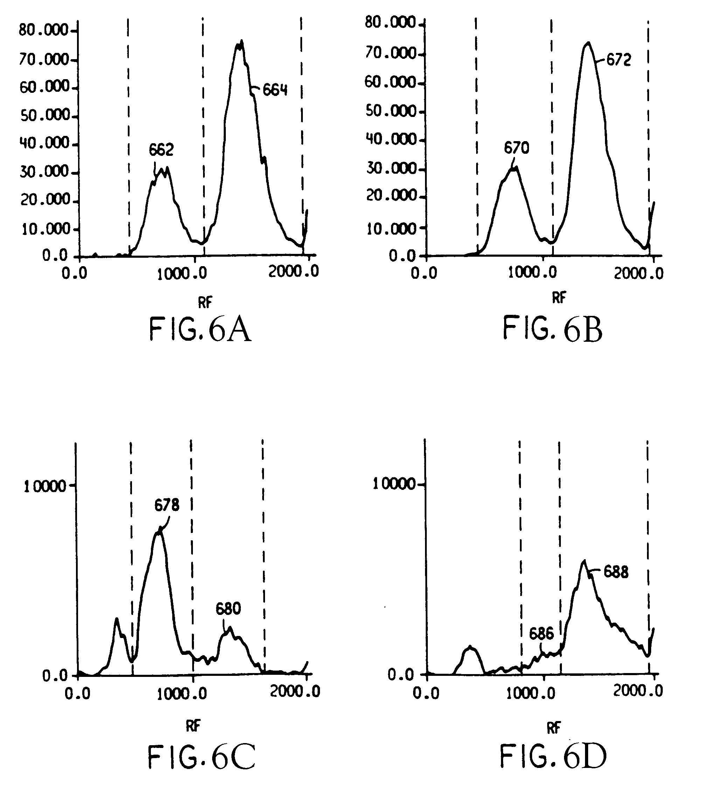

Referring now to FIGS. 5A-D and FIGS. 6A-D, a second two sets of one - dimensional scattergram multipart characterization results are illustrated, obtained from a second whole blood sample, again utilizing a prototype analyzing method similar to the analyzer instrument 540. The biological sample in each case again was a 28 microliter sample of whole blood, prepared for each channel as described with respect to FIGS. 3 and 5. The sample portion was lysed in the first channel 514 in the chamber 552 and then quenched before being fed to the analyzer 558. The results of analyzing utilizing a one-dimensional electronic sensing parameter are illustrated in FIGS. 5 and 6. DC was utilized to obtain the data in FIGS. 5A-5D and RF was utilized to obtain the data (for comparison) in FIGS. 6A-6D, utilizing the same sample portion and measuring at the same time in the analyzer 558. This results in two clearly identifiable data peaks 658 and 660 in FIG. 5A and peaks 662 and 664 in FIG. 6A. Peaks 658 and 662 are indicative of the percentage of L's and B's in the sample. Peaks 660 and 664 are indicative of the percentage of N's, E's and M's. Again, in one dimension, without further manipulation, the individual percentages are masked by the competing cells in the same data peak.

In the second channel 518, a second whole blood sample portion is combined with the magnetic microspheres with the N specific antibody bound thereto in the chamber 584, mixed, lysed and quenched and then placed in the magnetic field 592 before the mixture with the N's removed is fed to the analyzer 558. This results in two data peaks 666 and 668 in FIG. 5B and two data peaks 670 and 672 in FIG. 6B. Peaks 666 and 670 remain the same as the peaks 568 and 662 although their percentage of the sample mixture portion now is greater, while the peaks 668 and 672 are indicative of the percentage of the E's and M's with the N's removed. The data peaks 668 and 672 can then be compared with the respective data peaks 660 and 664 to determine the percentage of N's in the whole blood sample.

Next, or in any order including substantially simultaneously, a third sample portion is fed to the third channel 526 and mixed with the magnetic microspheres with the E and N specific antibody or antibodies bound thereto in the chamber 594, mixed, lysed and quenched and then placed in the magnetic field 602 before the mixture with the N's and E's removed is fed to the analyzer 558. This results again in two data peaks 674 and 676 in FIG. 5C and data peaks 678 and 680 in FIG. 6C. Peaks 674 and 678 again are the same contribution as the peaks 658 and 662, while the data peaks 676 and 680 are indicative of the percentage of the M's in the whole blood sample as compared to the data peaks 660 and 664. The data peaks 676 and 680 then can be compared to the data peaks 668 and 672 also to determine the percentage of E's in the whole blood sample.

A fourth sample portion is fed to the fourth channel 532 and mixed with the magnetic microspheres with a CD2 specific antibody bound thereto and the magnetic microspheres with a CD45R specific antibody bound thereto in the chamber 604, mixed, then placed in the magnetic field 612 and then the remaining mixture is removed to the holding chamber 644.

The N bound magnetic microspheres then are isolated in the chamber 604 by holding the magnetic microspheres with the N specific antibody bound thereto in the magnetic field 612 and removing the buffer solution. The sample portion then is returned to the chamber 604 after it is rinsed from the chamber 644 via a line 648 and mixed with the magnetic microspheres with the N specific antibody bound thereto, lysed and quenched and then again placed in the magnetic field 612 before the mixture with all the L's and N's removed is fed to the analyzer 558. This results in two data peaks 682 and 684 in FIG. 5D and two data peaks 686 and 688 in FIG. 6D. Peaks 682 and 686 represent only the B's, since all the L's have been removed to enhance the B's to a very significant data peak (i.e., amount of data). The B's are further enhanced since the N's also were removed from the peaks 684 and 688 and hence from the remaining sample portion.

The actual percentage calculations are performed as before as set forth hereinafter, with the data peak numbers calculated by dividing the data peak (number of cells counted in the peak) by the total data from both peaks (utilizing FIGS. 5A-5D for example purposes):

1) M% - determined from channel 526:

$$[\underline{(Pk1\ (658))}\ \underline{(Pk1\ (674))}]\ xx\ \underline{Pk2\ (676)} = M\%$$

2) M+E% - determined from channel 518:

$$[\underline{(Pk1\ (658))}\ \underline{(Pk1\ (666))}]\ xx\ \underline{Pk2\ (668)} = M+E\%$$

3)

$$E\% = M+E\% - M\%$$

4)

$$N\% = \underline{(Pk2\ (660))} - M+E\%$$

5) B% - determined from channel 532:

$$[\underline{(Pk2\ (668))}\ \underline{(Pk2\ (684))}]\ xx\ \underline{(Pk1\ (682))} = B^1\%$$

$$B^1\%\ xx\ [\underline{(Pk1\ (658))}\ \underline{(Pk1\ (666))}] = B\%$$

6)

$$L\% = \underline{(Pk1\ (658))} - B\%$$

For the data illustrated in FIGS. 5 and 6, the calculations from the RF and DC data are set forth in Table IV.

TABLE IV

| Channel | DC | | RF | |
|---|---|---|---|---|
| | Pk1 | Pk2 | Pk1 | Pk2 |
| 514 | 24.0 | 76.0 | 26.0 | 74.0 |
| 518 | 63.3 | 36.7 | 62.6 | 37.4 |
| 526 | 76.0 | 24.0 | 75.2 | 24.8 |
| 532 | 10.6 | 89.4 | 9.7 | 90.3 |

A full five part differential in percentage of the N's, L's, M's, E's and B's again was calculated from the DC and RF data peaks and again compared to a light sensing instrument for verification purposes, such as described in WO-A-8807198.

TABLE V

| 5 PART DIFFERENTIAL | | |
|---|---|---|
| LIGHT | DC | RF |
| N 60.37 | N 62.1 | N 58.5 |
| L 24.70 | L 22.4 | L 24.3 |
| M 8.62 | M 7.6 | M 8.6 |
| E 5.12 | E 6.3 | E 6.9 |
| B 1.18 | B 1.6 | B 1.7 |

The calculations are set forth below utilizing the DC data peaks:

1) $\quad M\% = \dfrac{24.0}{76.0}\ xx\ 24.0 = 7.6$

2) $\quad M+E\% = \dfrac{24.0}{63.3}\ xx\ 36.7 = 13.9$

3) $\quad E\% = (13.9) - (7.6) = 6.3$

4) $\quad N\% = (76.0) - (13.9) = 62.1$

5) $\quad B\% = \dfrac{36.7}{89.4}\ xx\ 10.6 = 4.4$

$$4.4 \text{ xx } \frac{24.0}{63.3} = 1.6$$

6)    L% = 24.0 - 1.6 = 22.4

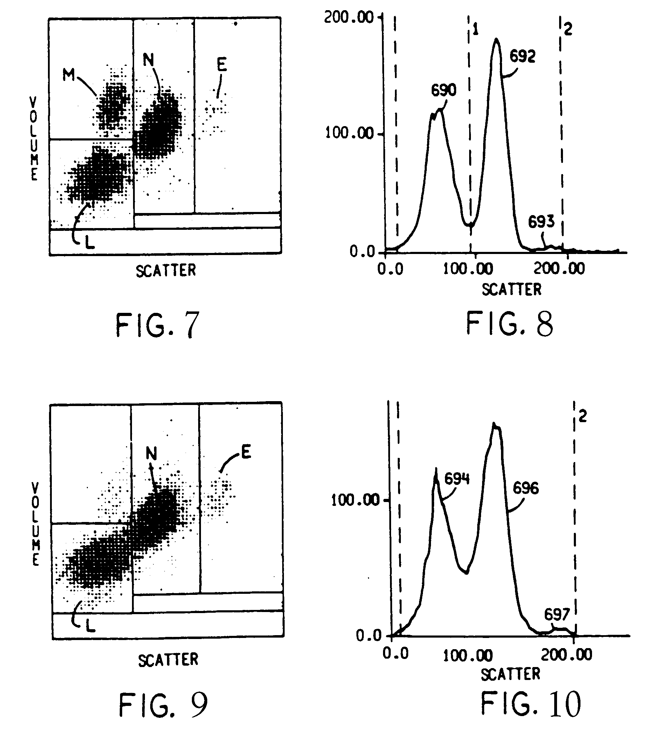

The data peaks in FIGS. 3-6 were depicted utilizing a single electronic sensing parameter, either DC or RF. Opacity was not illustrated, but also could be utilized if desired, since it is RF/DC as previously described. A single light sensing parameter also can be utilized, for example, median angle light scatter (Scatter), as illustrated in FIGS. 7-10.

Referring to FIG. 7, the M's, L's, N's and E's groups of cells are clearly separated by utilizing two parameters, Scatter and electrical volume. The B's are obscured in this data pattern. However, in one dimension, the same scatter data illustrated in FIG. 8 now results in the M's obscuring the L's or vice versa as seen in data peak 690. The N's and E's are illustrated in data peak 692 and the E's in data peak 693, representing the same data as in FIG. 7.

One method of solving the problem of the obscured data in the data peak 690 is to remove the M's. Currently this would be accomplished in an offline or prepreparation mode, since the M's are removed slowly when utilizing a CD14 specific antibody, such as M02 sold by Coulter Immunology Division of Coulter Corporation. For example, 400 microliters of a whole blood sample are combined with 200 microliters of a 2.5% solution of magnetic microspheres with the CD14 specific antibody bound thereto. The magnetic microspheres are first isolated by removing the fluid therefrom while holding the microspheres in a magnetic field. The sample then is added and the mixture is gently mixed, such as by rocking for about 30 minutes and then placed in a magnetic field for about 5 minutes after which the preprepared mixture, with the M's removed is analyzed in the instrument 540 as previously described. The data is illustrated again in two dimensions for comparison purposes in FIG. 9, where it clearly can be seen that the M's have been depleted by comparison with FIG. 7, enhancing the remaining WBC population data.

The one-dimensional scatter data is illustrated in FIG. 10, again resulting in two data peaks 694 and 696. The peak 694 is the data peak 690 with the M's removed, leaving only the L's. The peak 696 is the same as the data peak 692, and peak 697 is the same as peak 693, both peaks 696 and 697 are enhanced by the removal of the M's from the sample portion. Although not specifically illustrated, the sample mixture with the M's removed as illustrated in peak 694, now can be further depleted to perform L subset analyses, as will be further described hereinafter.

Although the analyzer 500 is illustrated for explanation purposes utilizing four separate channels, this only enhances the speed of the classification method of the invention. The invention also can be practised utilizing a single channel, with the different portions of the biological sample 502 being processed sequentially.

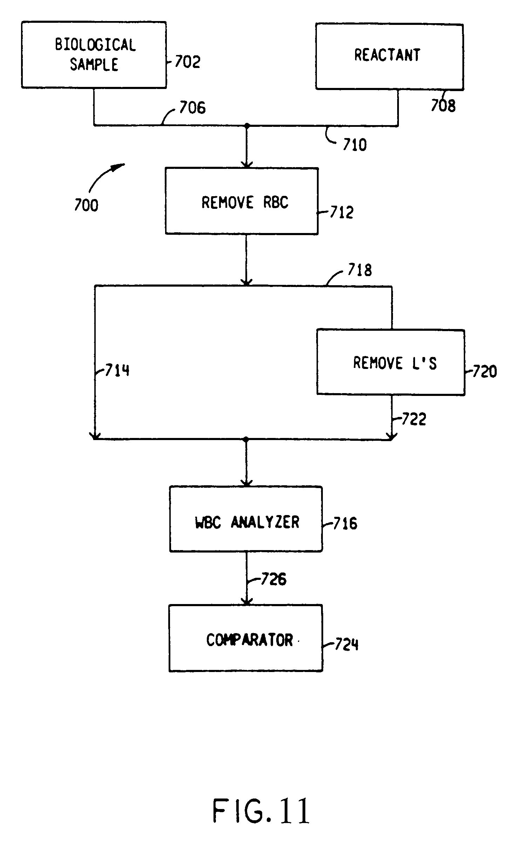

FIG. 11 illustrates an analyzer for enhancing small or obscure populations for classification thereof, designated generally by the reference numeral 700. This method of the invention can be utilized to determine such small populations as the B's or subsets of the L's. The analyzer 700 includes a biological sample 702, which contains at least a first set of viable biological cells (not illustrated), such as in or from a whole blood sample. If the classification is to be of B cells, then the sample 702 will include at least the B cell population and at least one other WBC population, such as the L cells. Generally the sample 702 would at least include all of the WBC populations, however, as previously described, various populations or subsets thereof can be eliminated offline or in a pre-preparation step. If the classification is to be of a WBC population subset, then the sample 702 will include at least one WBC population having at least one definable subset.

Describing first the classification of B cells, the B cells are a very small population of the total WBC populations, generally on the order of about less than one percent. Clearly, eliminating the WBC populations which obscure the B cells, will enhance the analysis and classification of the B's, because the B's then become a much greater and significant part of the remaining WBC population, as the other WBC populations are eliminated. Clearly, also, this enhancement will be true for all small or obscured cell populations of interest.

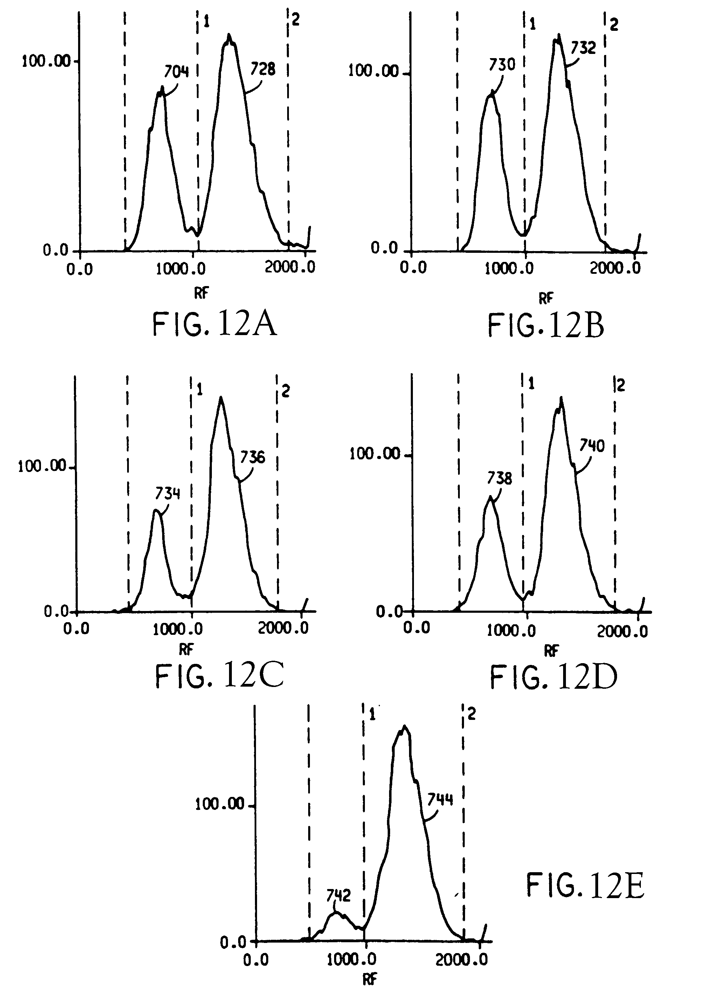

In analyzing the B cell populations utilizing a single measuring dimension or parameter, for example RF as illustrated in FIG. 12 to be described hereinafter, the B's are obscured by the L's in a single peak 704. The L's are around thirty (30) percent of the total WBC population and hence the B's still are only about three (3) percent even of only the L's and B's together. Therefore, to more definitely analyze and classify the B's, the L's are totally or substantially eliminated from the sample.

The sample 702 is combined via a line 706 with at least one reactant 708 via a line 710. The RBC's are removed in an RBC removal station 712 by one of the methods previously described. Once the RBC's are removed, a first portion of the resulting mixture is fed via a line 714 to a WBC analyzer 716. The WBC analyzer can be the same as those previously described, or minor variations thereof. The single sensing parameter can be electronic, such as RF or DC or light, such as Scatter or any other desired light parameter.

A second portion of the mixture is fed via a line 718 to a L removal station 720, wherein the L cells are bound to magnetic microspheres which include an L specific monoclonal antibody or antibodies bound thereto. The remainder of the mixture with at least the B cells remaining is fed via a line 722 to the WBC analyzer 716. The results of the two analyzed portions are fed to a comparator 724 via a line 726, where the two analyzed results are compared to determine the percentage of B's in the sample 702. The B's by this method have been made to change from about one to three percent of the sample mixture to substantially one hundred percent in the first data peak 704, providing definitive analysis and characterization. In a like manner, when the CD or other WBC population group chosen is small, all or some of the rest of the L's can be removed enhancing the analyzation of the remaining CD group of interest.

Next, describing the analysis and classification of one or more WBC population subsets, for example, the CD2, CD4 or CD8 WBC population subsets. The sample 702 again will include at least a first set of viable biological cells (not illustrated), such as in or from a whole blood sample. The sample 702 will include at least the WBC population subset of interest and at least one other obscuring WBC population or population subset and generally will include all the WBC populations.

The sample 702 again is fed to the RBC removal station 712 and then a first portion is fed to the WBC analyzer 716 via the line 714. A second portion will be utilized to determine the CD2 group, for example, and thus the second portion will be mixed with magnetic microspheres which include a CD2 specific monoclonal antibody bound thereto, such as T11 sold by Coulter Immunology Division of Coulter Corporation. The remainder of the mixture, with the CD2 cells now removed, will be fed via the line 722 to the WBC analyzer 716. The two analyzed results then are compared in the comparator 724 to provide the desired characterization of the CD2 group of cells.

The operation of the analyzer 700 can be performed on the instrument 540, utilizing the respective channels as desired or on a further multichannel instrument or again on a single channel instrument in a sequential fashion.

Referring now to FIGS. 12A-12E, the CD4, CD8 and CD2 subsets were determined as depicted in the one dimensional scattergram characterization results illustrated, utilizing a prototype analyzing method similar to the analyzer instrument 540. The biological sample in each case was a 28 microliter sample of whole blood. The sample was combined with 122 microliters of buffer solution for a control sample utilized in the channel 714 (which can be the channel 514 of the instrument 540). The results of a one dimensional electronic sensing parameter, here RF, was utilized to obtain the data for FIGS. 12A-E. The sample portion was lysed for 4 seconds with 300 microliters of the RBC preferential lyse above referenced (such as in the chamber 552) and then quenched with 120 microliters of quench before being fed to the analyzer 716 (such as the analyzer 558). This results in two clearly identifiable data peaks 704 and 728 in FIG. 12A. Peak 704 includes the B's and L's and all subsets thereof as a single peak. The B's are a small enough percentage so as to not effect the analyzation of the desired L subset, however, the value of the B's could be subtracted if desired.

A second control was utilized by combining a second whole blood sample portion of 28 microliters with 50 microliters of magnetic microspheres without any L or L subset specific antibody bound thereto and 72 microliters of buffer solution. The sample portion was lysed and quenched as before and then fed to the analyzer 716. This results in two data peaks 730 and 732 in FIG. 12B. The data peaks 730 and 732 are substantially identical to the peaks 704 and 728, hence the inclusion of the microspheres did not appear to have any deleterious effects on the analysis.

A third 28 microliter sample portion is fed to the channel 718 (which can be any one of the channels 518, 526 and 532 of the instrument 540), combined with 50 microliters of the magnetic microspheres with a CD4 specific antibody bound thereto and 72 microliters of the buffer solution. The CD4 specific antibody can be T4 sold by Coulter Immunology Division of Coulter Corporation. The sample portion was mixed for 60 seconds, lysed and quenched as before and then placed in a magnetic field for 30 seconds, before the mixture with the CD4 subset population removed is fed to the analyzer 716. This results in two data peaks 734 and 736 in FIG. 12C. The peak 734 represents the L's without the CD4 subset population and then is compared to the peak 704 by the comparator 724 to obtain the percentage of the CD4 subset population in the sample.

Again, for testing and evaluation purposes, the same blood sample was analyzed in the above manner to obtain four sets of data peaks, one of which is actually depicted in FIGS. 12A-E. The data peaks in each set of data were substantially the same and hence a single set of data can be utilized. The data was averaged over the four sets of data to obtain the results. The average CD4 subset population percentage obtained was 45.7, which was compared with a light sensing instrument for verification purposes, such as the EPICS@ flow cytometer available from Coulter Corporation, which provided a percentage of 47.6.

A fourth 28 microliter sample portion is fed to the channel 718, combined with 50 microliters of the magnetic microspheres with a CD8 specific antibody bound thereto and 72 microliters of the buffer solution. The CD8 specific antibody can be T8 sold by Coulter Immunology Division of Coulter Corporation. The sample portion was again mixed, lysed and quenched and placed in a magnetic field as before. The mixture with the CD8 subset population removed then is fed to the analyzer 716. This results in two data peaks 738 and 740 in FIG. 12D. The data peak 738 represents the L's without the CD8 subset population, which then is compared to the peak 704 by the comparator 724 to obtain the percentage of the CD8 subset population in the sample. The average CD8 subset population percentage obtained was 25.0, which was compared with the light sensing instrument for verification purposes, which provided a percentage of 26.0.

A fifth 28 microliter sample portion is fed to the channel 718, combined with 50 microliters of the magnetic microspheres with a CD2 specific antibody bound thereto and 72 microliters of the buffer solution. The CD2 specific antibody can be T11 sold by Coulter Immunology Division of Coulter Corporation. The sample portion was again mixed, lysed and quenched and placed in a magnetic field as before. The mixture with the CD2 subset population removed then is fed to the analyzer 716. This results in two data peaks 742 and 744 in FIG. 12E. The data peak 742 represents the L's without the CD2 subset population, which then is compared to the peak 704 by the comparator 724 to obtain the percentage of the CD2 subset population in the sample. The average CD2 subset population percentage obtained was 82.7, which was compared with the light sensing flow cytometer instrument, which provided a percentage of 79.0.

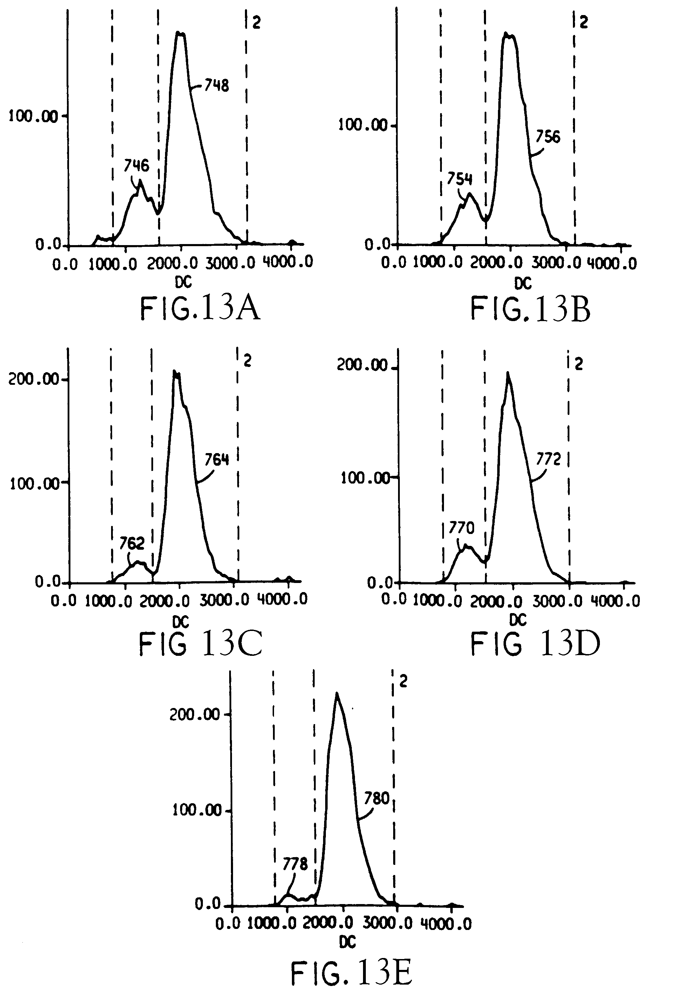

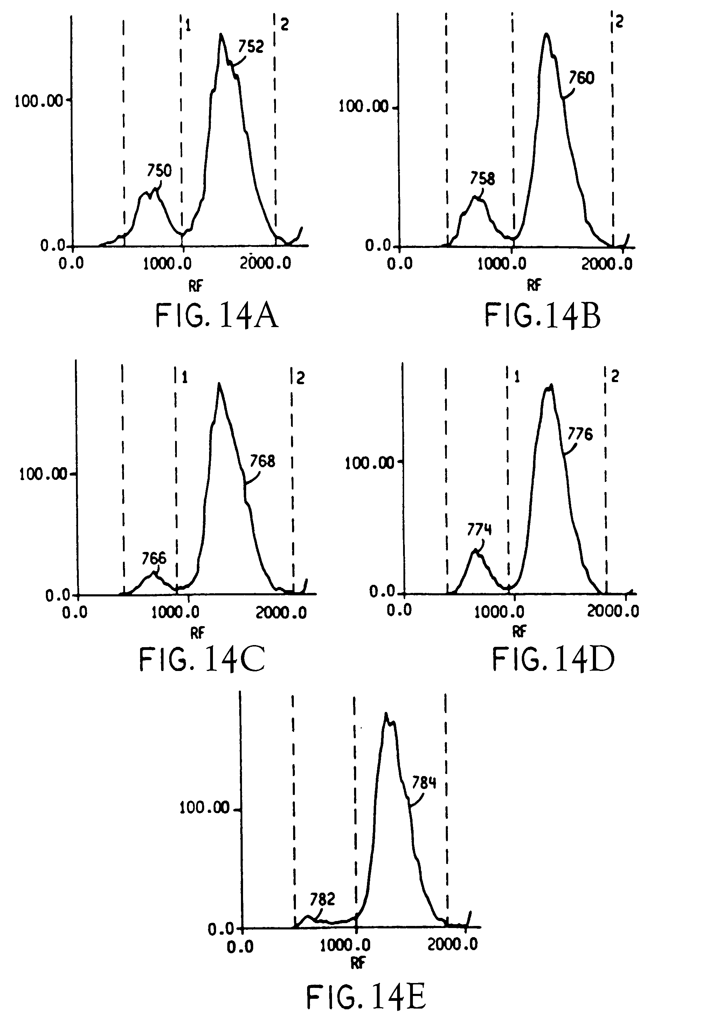

The results of another subset analysis utilizing a one-dimensional electronic sensing parameter of CD4, CD8 and CD2 subset populations is illustrated in FIGS. 13A-E and 14A-E. DC was utilized to obtain the data in FIGS. 13A-E, while RF was utilized to obtain the data in FIGS. 14A-E, utilizing the same sample portion and measured at the same time.

The sample portion was lysed and quenched before being fed to the analyzer 716 in substantially the same manner as described with respect to FIGS. 12A-E. In the case of FIGS. 13A and 14A, only a buffer solution was added and in the case of FIGS. 13B and 14B, a buffer solution as well as magnetic microspheres without the L or L subset specific antibodies were combined with the sample. The control without microspheres resulted in data peaks 746 and 748 in FIG. 13A and data peaks 750 and 752 in FIG. 14A. Peaks 746 and 750 are representative of the L's and B's in the sample. The control with control microspheres combined with the sample, but with the microspheres removed therefrom utilizing the magnetic field, resulted in data peaks 754 and 756 in FIG. 13B and data peaks 758 and 760 in FIG. 14B.

A third whole blood sample portion is combined with 50 microliters of magnetic microspheres with a CD4 specific antibody bound thereto. The mixture is mixed, lysed, quenched and placed in the magnetic field to remove the CD4 subset population for the sample portion mixture before it is fed to the analyzer 716. The DC analysis results in two data peaks 762 and 764 in FIG. 13C, while the RF analysis results in two data peaks 766 and 768 in FIG. 14C. The data peaks 746 and 762 are compared as are the data peaks 750 and 766 to obtain the percentage of the CD4 subset population in the sample.

In FIGS. 13 and 14, the data peaks are one of three separate sample sets from the same whole blood sample which were averaged to obtain the results. In the case of CD4, the percentage obtained from DC was 57.0, RF was 57.1 and the light sensing flow cytometer instrument was 54.6.

To obtain the CD8 subset population analysis, a fourth whole blood sample portion was combined with 50 microliters of magnetic microspheres with a CD8 subset population specific antibody bound thereto. The sample portion is mixed, lysed, quenched and held in a magnetic field to remove the CD8 subset population from the mixture, before the mixture is fed to the analyzer 716. The DC analysis results in two data peaks 770 and 772 in FIG. 13D, while the RF analysis results in two data peaks 774 and 776 in FIG. 14D.

The data peaks 746 and 770 are compared as are the data peaks 750 and 774 to obtain the percentage of the CD8 subset population in the sample. In the case of CD8, the percentage obtained from DC was 17.4, from RF was 18.6 and from the light sensing flow cytometer instrument was 17.7.

To obtain the CD2 subset population analysis, a fifth whole blood sample portion was combined with 50 microliters of magnetic microspheres with a CD2 subset population specific antibody bound thereto. The sample portion is mixed, lysed, quenched and held in a magnetic field to remove the CD2 subset population from the mixture, before the mixture is fed to the analyzer 716. The DC analysis results in two data peaks 778 and 780 in FIG. 13E, while the RF analysis results in two data peaks 782 and 784 in FIG. 14E.

The data peaks 746 and 778 are compared as are the data peaks 750 and 782 to obtain the percentage of the CD8 subset population in the sample. In the case of CD8, the percentage obtained from DC was 79.6, from RF was 79.3, and from the light sensing flow cytometer instrument was 74.7.

The B and L subset population analysis referred to with respect to FIGS. 11-14 was obtained utilising a one-dimensional electronic sensing parameter. A light sensing parameter also could be utilised.

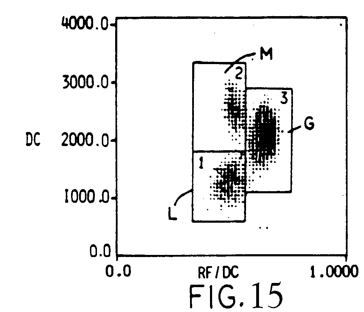

By handling sample portions in the same manner as those utilised to obtain the data shown in FIGS. 12-14, FIG. 15 illustrates the results of a control, without microspheres. Clearly, the normal blood sample L, M and G grouping is totally obscured, and there is just is one, unidentifiable data grouping.

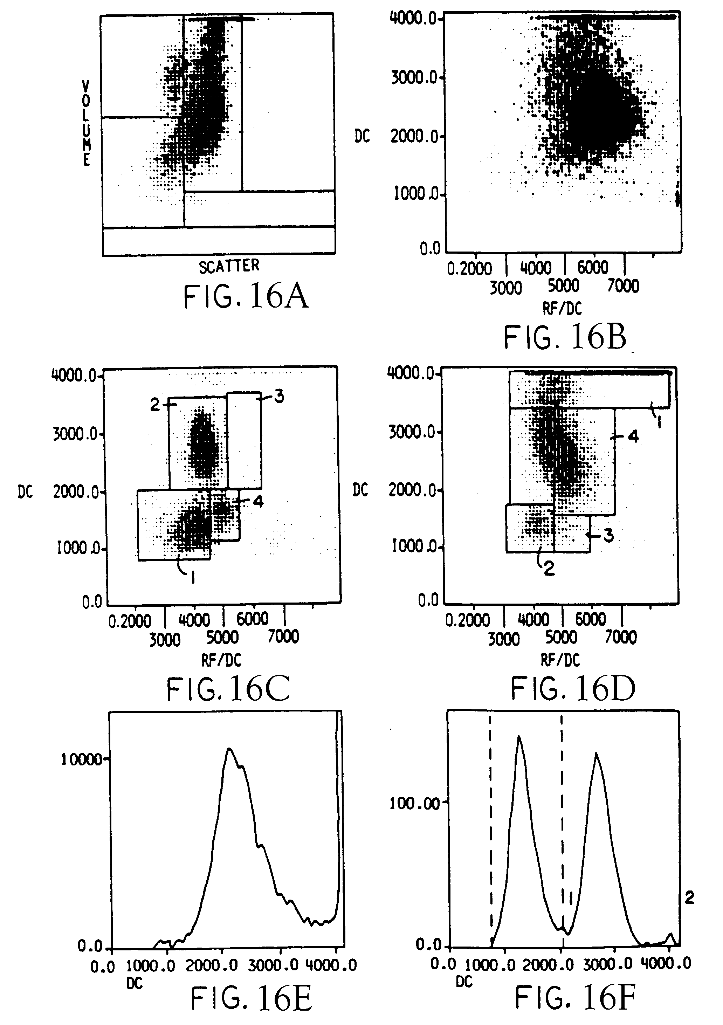

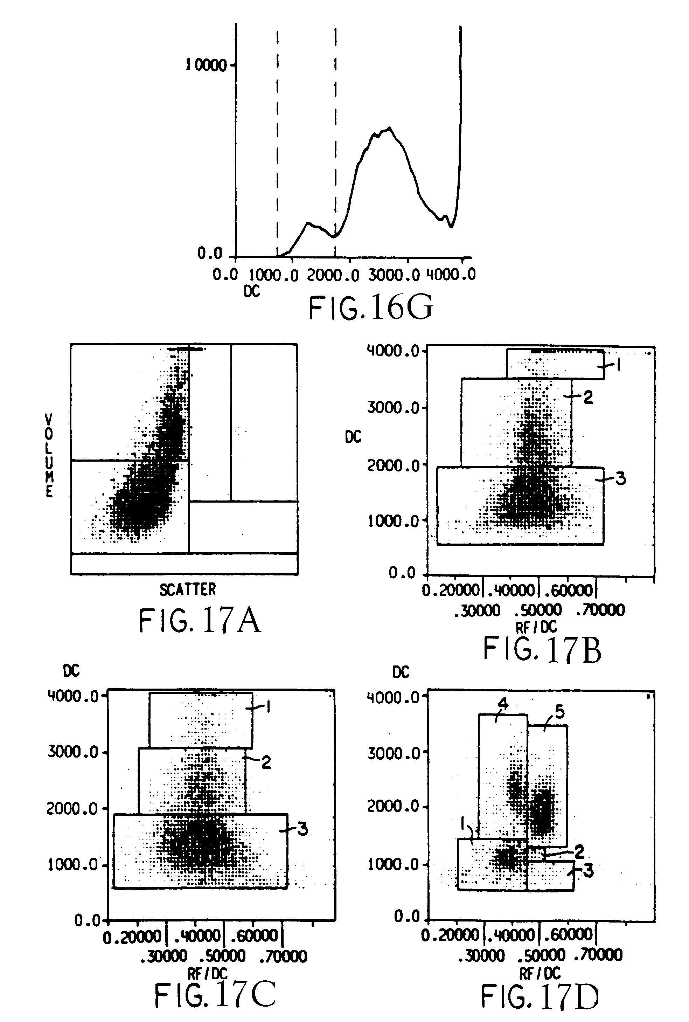

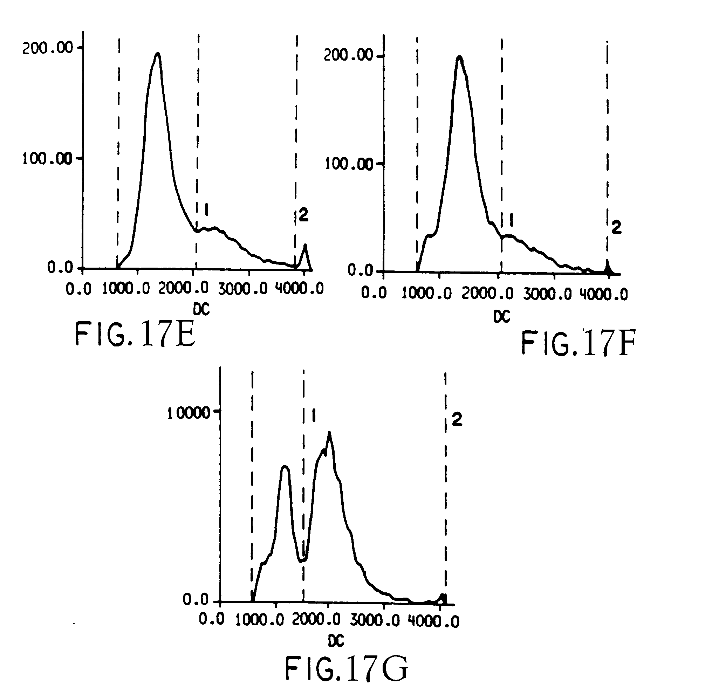

FIGS. 16 and 17 illustrate the results of analysing two abnormal whole blood samples. The first sample depicted in FIGS. 16A-G was analysed and displayed in several different manners. FIGS. 16A and 17B depict two different two-dimensional sensing histograms of the whole blood sample, one with light sensing and one with only electronic sensing, without any treatment of the blood sample.

The results of two different treatments of the abnormal whole blood sample are illustrated in FIGS. 16C and 16D. In FIG. 16C, a 200 microliter portion of the sample was combined with 200 microliters of magnetic microspheres having a N and E specific antibody bound thereto. The mixture is mixed, lysed, quenched and held in a magnetic field while the remaining portion is removed and fed to the analyzer without the N and E bound cells therein. It appears clear that a substantial portion of the abnormal cells have been removed, since the pattern in FIG. 16C has become very much more defined than the pattern in FIG. 16B.

In the second treatment, a 200 microliter portion of the sample was combined with 200 microliters of magnetic microspheres having only a N specific antibody bound thereto. Although some of the cells have been removed, indicating that some are bound to the N antibody, a significant portion of the abnormal cells remain, especially as seen at the top of the histogram. It would therefore appear that some of the abnormal or diseased cells bind to the N+E antibody. This type of depletion treatment can be utilized for diagnosis and treatment of particular diseases.

The data histograms of FIGS. 16A-D were developed utilizing two - dimensional sensing. The same information can be developed utilizing a single sensing parameter, for example, a single electronic sensing parameter as depicted in FIGS. 16E-16G. The one - dimensional sensing, here DC, produces the histogram depicted in FIG. 16E when the sample is analyzed without any treatment. The single data peak and the data at the far right of the histogram are clear indications of an abnormal sample.

Again, the same treatment was utilized as referred to above with respect to FIGS. 16C and 16D and, in fact, the one-dimensional sensing data was generated at the same time as the two-dimensional sensing data. The analyzing instrument can, as described above, include multiple sensing parameters or only one single parameter. Again, the removal of the N and E bound cells produces a histogram in FIG. 16F, which again illustrates that most of the abnormal cells have been removed. The histogram in FIG. 16G illustrates again that a significant number of the abnormal cells have not been removed.

The results of analyzing the second abnormal whole blood sample are illustrated in FIGS. 17A-G. The results of analyzing the sample without treatment are illustrated in two dimensional histograms in FIGS. 17A and 17B. Clearly, a normal whole blood sample data pattern is not seen. The histogram of FIG. 17B prepared in the instrument, can be compared to the histogram in FIG. 17C prepared offline, which do not appear significantly different. A 28 microliter sample portion was prepared offline or preprepared before the analysis depicted in FIG. 17C.

A portion of the sample was depleted of the CDS subset population and then analyzed to provide the histogram in FIG. 17D. A 28 microliter portion of the whole blood sample was combined with 122 microliters of magnetic microspheres having a CD5 specific antibody bound thereto, such as T1 sold by Coulter Immunology Division of Coulter Corporation. The mixture was mixed, lysed, quenched and held in a magnetic field to remove the CD5 bound cells. This clearly removed a significant portion of the abnormal cells as can be seen by comparing FIGS. 17B or FIG. 17C with FIG. 17D. The histograms of FIGS. 17A-D were made with two-dimensional sensing, while the same data is depicted in one-dimension histograms in FIGS. 17E-G.

Again, no treatment was performed on the online sample depicted in FIG. 17E or on the offline sample depicted in FIG. 17F and the CD5 subset population was depleted from the sample depicted in FIG. 17G.

As discussed hereinbefore, the overlapping populations of cells can be of interest for diagnostic as well as treatment of diseases of the blood. For example, some immature cells express both CD4 and CD8 receptors. The obtaining of an analysis of the overlapping percentage of cell subset populations has not been available when utilizing electronic sensing parameters or a minimal number of light parameters or simplified combinations thereof. For example, in some light sensing instruments, three sensing parameters are utilized to obtain the overlapping data, both forward and 90° light scatter and fluorescence. One example of overlapping populations in a normal whole blood sample is the CD2 and CD8 subset populations. An abnormal overlapping of populations is found in CLL (chronic lyphocytic leukemia). In the CLL disease state, the CD5 and CD20 subset populations overlap.

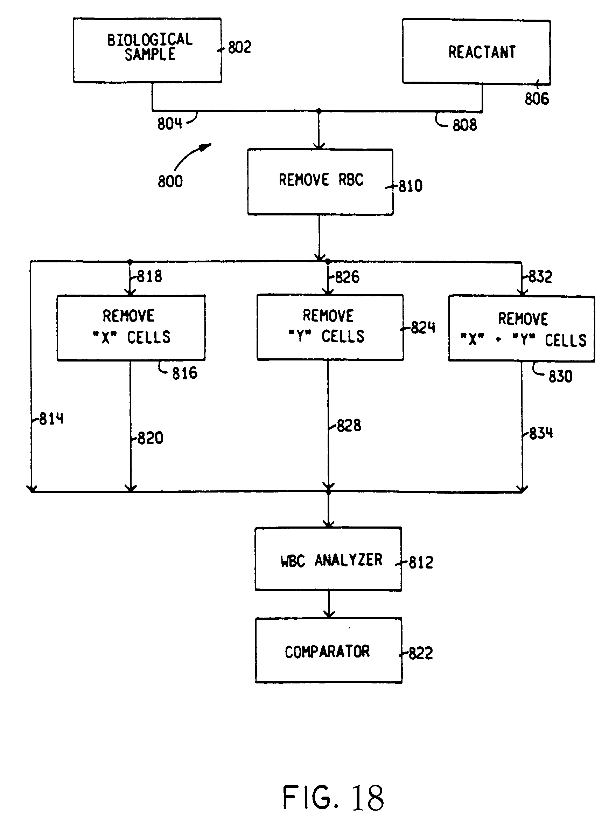

Referring to FIG. 18, apparatus for performing an overlapping classification of cells is designated generally by the reference numeral 800. The instrument or analyzer 800 includes a biological sample 802, which contains at least a first set of viable biological cells (not illustrated), including at least two overlapping white blood cell populations or subset populations, such as in or from a whole blood sample.

The cells of the biological sample 802 are to be involved in a biological reaction in a quantitative and/or qualitative determination or analysis. The biological sample 802 can include a buffer into which the cells are added.

The biological sample 802 is combined via a line 804 with at least one reactant 806 via a line 808. In the analyzer 800, the RBC's are removed from the mixture at an RBC removing station 810. As stated in the first parent application, the RBC's can be removed from the station 810 in a number of ways, such as enumerated with respect to the station 20.

A first portion of the mixture with the RBC's removed, then is fed to a WBC analyzer 812 via a line 814. This obtains a standard or control for the total or whole WBC populations of the biological sample 802. The analyzer 812 can be the same as the analyzer 86, illustrated and described in WO-A-8807199 or can be a light sensing analyzer, such as described in WO-A-8807198. The single sensing parameter can be electronic, such as RF or DC or light, such as median angle light scatter (Scatter) or any other desired light parameter.

A second portion of the mixture is fed to an "X" removing station 816 via a line 818. The station 816 removes a first overlapping population of cells "X". The X's are removed or depleted by the addition of the appropriate magnetic microspheres with an X specific antibody bound thereto. A magnet or magnetic field is utilized, as before discussed, to remove the magnetically bound cells from the mixture. The remaining mixture with the X's removed then is fed via a line 820 to the analyzer 812. The analyzed results of the "X" removed portion of the mixture than can be compared with the analyzed results of the first mixture portion in a comparator 822 to obtain the percentage of X's in the biological sample 802.

A third portion of the mixture is fed to a second removing station 824 via a line 826. The station 824 removes a second overlapping population of cells "Y". The Y's are removed by the addition of appropriate magnetic microspheres with a Y specific antibody bound thereto. The remaining mixture with the Y's removed then is fed via a line 828 to the analyzer 812. The analyzed results of the "Y" removed portion of the mixture then is compared to the results of the first and second mixture portions in the comparator 822 to verify if there is an overlapping of the X and Y populations in the biological sample 802. This operation verifies that the X and Y populations overlap, only if the X and Y populations or population ratios are generally known or if the total of the X and Y depleted populations is greater than 100%.

In most cases, a fourth portion of the mixture is fed to a removing station 830 via a line 832. The station 830 removes both the X and Y populations (X+Y). The X's and Y's are removed by the addition of the appropriate magnetic microspheres with the X and Y specific antibodies bound thereto. The remaining mixture with the X's and Y's removed then is fed via a line 834 to the analyzer 812. The analyzed results of the "X" + "Y" removed portion of the mixture then can be compared to the results of the other mixture portions to obtain the percentage of overlapping of the X and Y populations in the biological sample 802.

Thus, the analyzer 800 can perform a single overlapping classification of cells, utilizing lines or channels 814, 818 and 826 and a full percentage overlapping classification utilizing all four lines. One most important feature of the analyzer 800 is that the mixtures can be analyzed utilizing only a single electronic or light parameter. Other combinations can be utilized, but in each case only a single sensed parameter or characteristic is necessary to perform the overlapping classification of the invention. (The analysis also can be obtained with multiple sensing parameters.)

A specific analyzing instrument embodiment incorporating the method and apparatus of the analyzer 800 is not illustrated, however, one such instrument can be the instrument 540. Again, the overlapping method and apparatus of the invention can be practiced on only a single channel of the instrument 540 or on a single channel instrument, not illustrated.

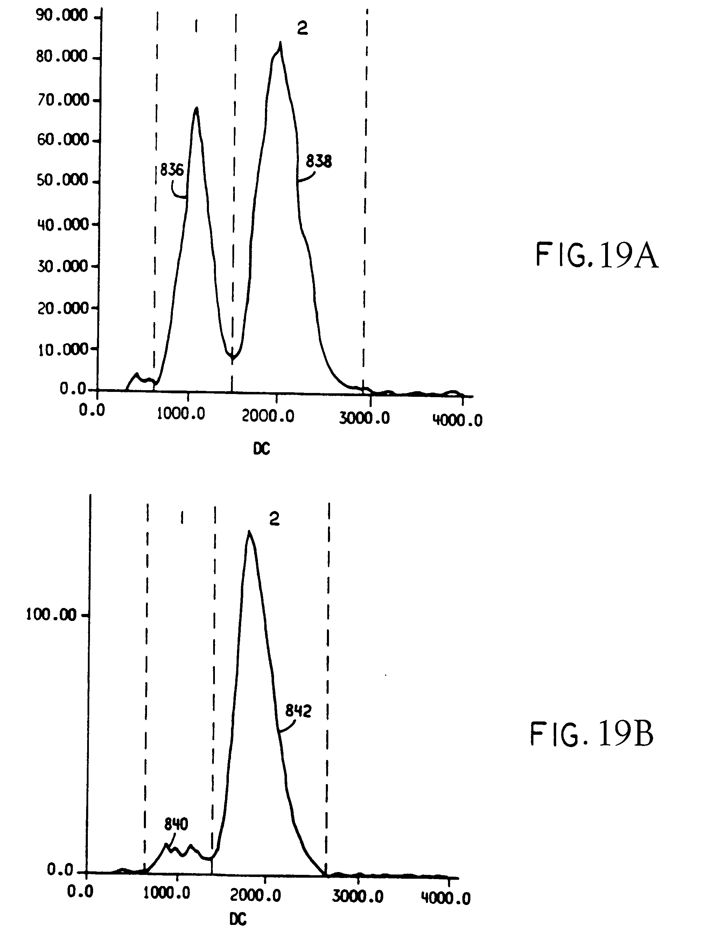

Referring now to FIGS. 19A-19D, one set of one-dimensional scattergram overlapping characterization results are illustrated, obtained from a whole blood sample, utilizing a prototype analysis method similar to the analyzer instrument 540 and described with respect to the instrument 800. The biological sample in each case was a 28 microliter sample of whole blood, which was combined with 122 microliters of buffer solution utilized in the first channel 814. The sample portion was lysed with 300 microliters of the above referenced RBC preferential lyse for 4 seconds and then quenched with 120 microliters of quench before being fed to the analyzer 812.

The data results of analyzing the portion with a one-dimensional electronic sensing parameter, here DC, is illustrated in the histogram of FIG. 19A. The data results in two clearly identifiable data peaks 836 and 838. As before, the peak 836 is indicative of the percentage of L's and B's in the sample, while the peak 838 is indicative of the percentage of N's, E's and M's.

The sample was then treated to deplete first the X and then the Y cell populations as above referenced. In this case, the X population was the CD2 subset population of the L's and the Y population was the CD20 subset population of the L's. A second portion of the sample was fed to the station 816, wherein 60 microliters of magnetic microspheres having a CD2 specific antibody bound thereto was combined with the sample portion, mixed, lysed, quenched and then held in a magnetic field to remove the CD2 subset population. The remaining mixture then was fed to the analyzer 822 resulting in two data peaks 840 and 842 in FIG. 19B. The peak 840 is the remaining L's, which then is compared in the comparator 822 to the peak 836 to obtain the percentage of the CD2 subset population in the sample.

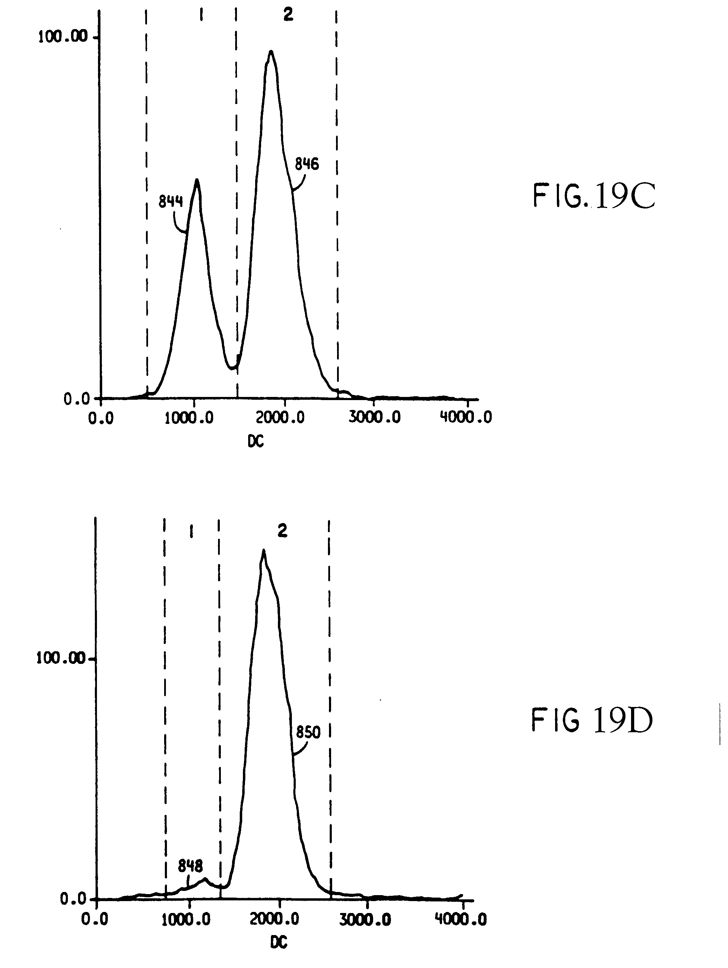

A third portion of the sample is fed to the station 824, wherein 50 microliters of magnetic microspheres having a CD20 specific antibody bound thereto was combined with the sample portion, mixed, lysed, quenched and then held in a magnetic field to remove the CD20 subset population. The remaining CD20 removed mixture then was fed to the analyzer 822 resulting in two data peaks 844 and 846 in FIG. 19C. The peak 844 is the remaining L's, which then is compared to the peak 836 to obtain the percentage of the CD20 subset population in the sample.

If the normal relative percentages of the CD2 and CD20 subsets are known, then this can be sufficient to identify that the subset populations are overlapping. Also, if the two subset population percentages add to a total of greater than 100 percent, then clearly, this also indicates that the two subset populations overlap. In cases of small overlapping percentages, it then is important to obtain the percentage of overlapping subset populations to verify that the subset populations do overlap. A further depletion is necessary to obtain the overlapping percentage.

A fourth portion of the sample is fed to the station 830, wherein magnetic microspheres having the CD2 specific antibody and magnetic microspheres having the CD20 specific antibody bound thereto are combined to remove both the CD2 and CD20 subset populations. The remaining CD2 and CD20 depleted mixture then was fed to analyzer 822 resulting in two data peaks 848 and 850 in FIG. 19D. The peak 848 again is the remaining L's, which then is compared to the peak 836 to obtain the percentages of subset populations removed by the CD20 and CD2 specific antibodies. This result then is compared to the individual CD2 and CD20 results to obtain the overlapping percentages, if any.

The exact overlapping percentages are calculated as follows:

I. Subset "A" (CD2)

(a) $\frac{\text{(Data Peak 838)}}{\text{(Data Peak 842)}}$ xx (Data Peak 840) = A'

(b) $\frac{\text{(Data Peak 836) - A'}}{\text{(Data Peak 836)}}$ = % Subset A (CD2)

II. Subset "B" (CD20)

(a) $\frac{\text{(Data Peak 838)}}{\text{(Data Peak 846)}}$ xx (Data Peak 844) = B'

(b) $\frac{\text{(Data Peak 836) - B'}}{\text{(Data Peak 836)}}$ = % Subset B (CD20)

III. Subset "A+B" (CD2 + CD20)

(a) $\frac{\text{(Data Peak 838)}}{\text{(Data Peak 850)}}$ xx (Data Peak 848) = A'+B'

(b) $\frac{\text{(Data Peak 836) - A'+B'}}{\text{(Data Peak 836)}}$ = % of Subset A+B

IV. Overlapping Portion

[Subset "A" + Subset "B") - [Subset "A+B"] = overlap

The results (averaged from two separate preparations) are illustrated in Table VI as follows:

TABLE VI

|          | Peak | Relative Percentage | Peak | Relative Percentage |
|----------|------|---------------------|------|---------------------|
| Control  | 836  | 35.7                | 838  | 64.3                |
| CD2      | 840  | 7.1                 | 842  | 92.9                |
| CD20     | 844  | 33.6                | 846  | 66.4                |
| CD2+CD20 | 848  | 3.5                 | 850  | 96.5                |

The relative percentages in Table VI are the percentage of the two peaks of the total percentage of 100. The percentage of CD2 was calculated as 86.2, CD20 was 8.9 and CD2+CD20 was 93.5. Therefore, the overlapping percentage was (CD2+CD20) - CD2+CD20 = (86.2 + 8.9) - 93.5 = 1.6.

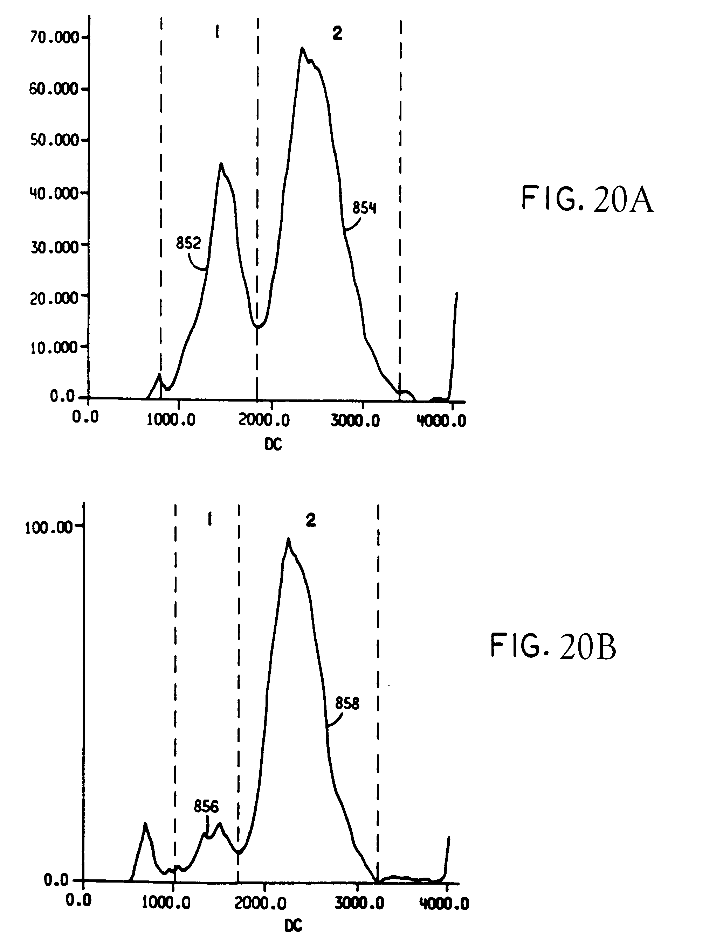

A second sample was depleted to obtain the percentage overlap of the CD2 and CD8 subset populations as illustrated in FIGS. 20A-D. Again, a first portion of the sample was fed to the channel 814 without a depletion to obtain the control data illustrated in FIG. 20A. The data results in two clearly identifiable data peaks 852 and 854. The peak 852 again is indicative of the percentage of L's and B's in the sample.

A second sample portion is depleted of the CD2 subset population as before described in the channel 818, resulting in two data peaks 856 and 858 in FIG. 20B. The remaining L peak 856 again is compared to the control peak 852 to obtain the percentage of the CD2 subset population in the sample.

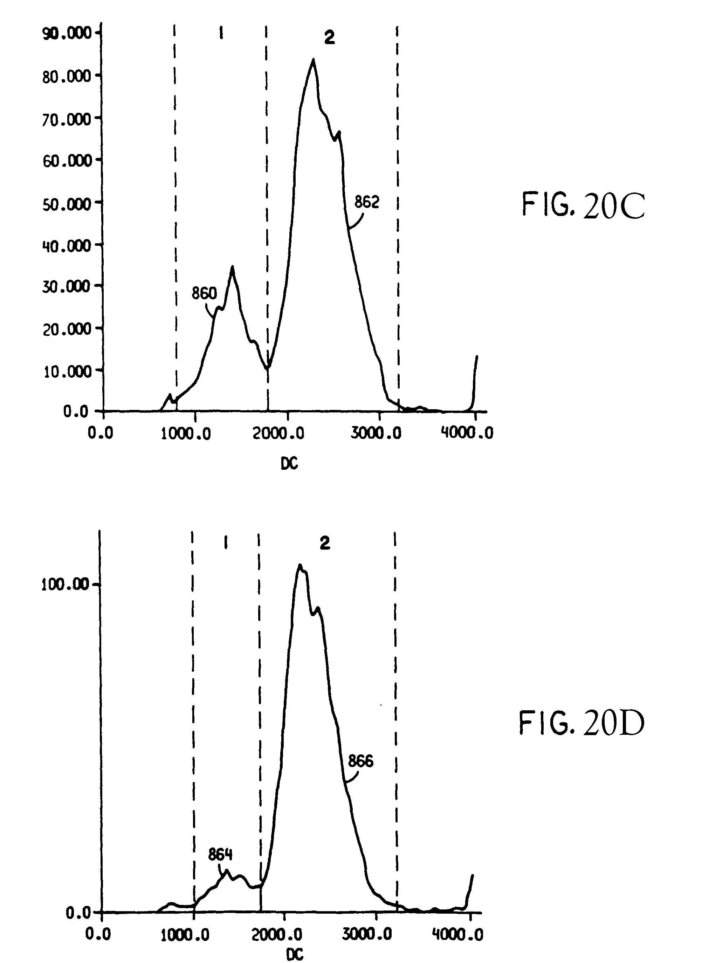

A third sample portion is depleted of the CD8 subset population in the channel 826, resulting in two data peaks 860 and 862 in FIG. 20C. The peak 860 is compared to the control peak 852 to obtain the percentage of the CD8 subset population in the sample.

A fourth sample portion is depleted of the CD2+CD8 subset populations in the channel 832, resulting in two data peaks 864 and 866 in FIG. 20D. The peak 864 is compared to the control peak 852 to obtain the percentage of CD2+CD8 subset populations in the sample.

The results are illustrated in Table VII as follows:

TABLE VII

|          | Peak | Relative Percentage | Peak | Relative Percentage |
|----------|------|---------------------|------|---------------------|
| Control  | 852  | 30.3                | 854  | 69.7                |
| CD2      | 856  | 9.0                 | 858  | 91.0                |
| CD8      | 860  | 22.1                | 862  | 77.9                |
| CD2+CD8  | 864  | 7.6                 | 866  | 92.4                |

The percentage of CD2 was calculated as 77.3, CD8 was 34.7 and CD2+CD8 was - 81.1. Therefore, the overlapping percentage was (77.3 + 34.7) - 81.1 = 30.9.

Also, although the method of the invention has been described utilizing whole blood samples, there can be instances where it is desired to utilize a portion of a sample with the RBC's and/or some of the WBC populations removed. Clearly, the RBC's are still removed, but arguably externally and not within the apparatus used in the invention. Such removal or prepreparation can be carried out in numerous conventional ways, such as utilizing a lysing reagent, density or centrifugation techniques, such as ficoll, dextran, "buffycoat", etc. In an automated analyzer utilizing the invention, it would be preferable to utilize a whole blood sample for speed and integrity in the analysis of the sample.

Many modifications and variations of the present invention are possible in light of the above teachings. The volumes of microspheres specified are stated in weight of microspheres per volume of diluent. Although volumes on the order of about 20 microliters of sample, such as a whole blood sample, have been utilized for example purposes herein, smaller or larger sample volumes also can be utilized as desired. For example, as small as about 2 microliters of a sample up to whatever volume of sample is practical for the particular instrument or technique can be utilized. Although some of the examples were performed in sequential steps, the steps can also be performed simultaneously. A simultaneous analysis allows the least complex instrument module to be utilized.

## Claims

1. A method of determining the percentage of at least one white blood cell (WBC) population or subset population in a whole blood sample, which comprises:

   analysing first and second portions of the sample, after removing the red blood cell population without significantly adversely affecting relative qualities and/or quantities of WBC populations and/or subset populations, to determine their respective WBC population or subset population characteristics; and comparing the two analysed characteristics;

   wherein the second portion has been treated by substantially depleting at least one WBC population or subset population thereof, by mixing therewith microspheres having bonded thereto a monoclonal antibody specific to the at least one WBC population or subset population; and wherein each analysing step is by Coulter sensing or light-scattering techniques, and characterised by utilising a single electronic or light parameter.

2. The method according to claim 1, wherein the microspheres are magnetic, and the depletion comprises removing at least part of the second portion while the magnetic microspheres are held in a magnetic field.

3. The method according to claim 1 or claim 2, wherein the at least one WBC population or subset depleted from the second portion comprises its neutrophil population.

4. The method according to any preceding claim, further comprising subtracting at least two WBC populations or subset populations from a third portion of the whole blood sample;

   analysing the third portion, after removing the red blood cell population, to determine at least one WBC population or subset population characteristic of the whole blood sample; and

   comparing the three analysed characteristics, to determine the percentage of at least two WBC populations or subset populations of the whole blood sample.

5. The method according to claim 4, wherein the WBC populations or subset populations are removed from the third portion by mixing therewith microspheres having bonded thereto a monoclonal antibody specific to the WBC populations or subset populations, and removing at least part of the third portion while the magnetic microspheres are held in a magnetic field.

6. The method according to claim 4 or claim 5, wherein the at least two WBC populations or subset populations subtracted from the third portion comprise the neutrophil and eosinophil populations.

7. The method according to claim 4 or claim 5, wherein the at least two WBC populations or subset populations subtracted from the third portion comprise the lymphocyte and neutrophil populations.

8. The method according to any of claims 4 to 7, further comprising subtracting at least two WBC populations or subset populations from a fourth portion of the whole blood sample;

   analysing the fourth portion, after removing the red blood cell population, to determine at least one WBC population or subset population characteristic of the whole blood sample; and
comparing the four analysed characteristics, to determine the percentage of at least three WBC populations or subset populations of the whole blood sample.

9. The method according to claim 8, comprising comparing at least four analysed WBC population characteristics, to calculate or determine the percentage of at least the eosinophil, monocyte, lymphocyte, basophil and neutrophil WBC populations of the whole blood sample, to perform a five-part WBC differential.

10. The method according to any preceding claim, wherein the at least one WBC population or population subset depleted from the second portion includes at least one WBC population or population subset which would otherwise obscure the analysis of the at least one WBC population or subset population of interest.

11. The method according to claim 10, further comprising subtracting at least the lymphocyte population from the first portion, prior to analysing it, to obtain a basophil percentage population of the whole blood sample.

12. The method according to claim 10, further comprising subtracting at least one WBC subset population of interest from the first portion, prior to analysing it, to obtain a percentage population of the WBC subset population of interest.

13. The method of any preceding claim, wherein each analysing step is electronic, utilising a single electronic parameter.

14. The method according to any of claims 1 to 12, wherein each analysing step is optical, utilising a single light parameter.

**Patentansprüche**

1. Verfahren zum Bestimmen des Prozent-Anteils mindestens einer weißen Blutzell(WBC)-Population oder Unterpopulation in einer Vollblutprobe, wobei das Verfahren aufweist:

Analysieren eines ersten und zweiten Anteils der Probe nach Entfernen der roten Blutzellpopulation ohne wesentliches nachteiliges Beeinträchtigen relativer Qualitäten und/oder Quantitäten der WBC-Populationen und/oder Unterpopulationen, um ihre jeweiligen WBC-Populations- oder Unterpopulationscharakteristika zu bestimmen; und Vergleichen der zwei analysierten Charakteristika;
wobei der zweite Anteil durch wesentliches Entfernen mindestens einer WBC-Population oder Unterpopulation davon behandelt worden ist, und zwar durch Dazumischen von Mikrokügelchen mit einem daran gebundenen monoclonalen Antikörper, der für mindestens eine WBC-Population oder Unterpopulation spezifisch ist; und wobei jeder Analyseschritt durch Coulter-Abtastung oder Lichtstreutechniken durchgeführt wird und durch Verwenden eines einzelnen elektronischen oder Lichtparameters charakterisiert ist.

2. Verfahren nach Anspruch 1, wobei die Mikrokügelchen magnetisch sind und das Entfernen das Wegnehmen mindestens eines Teiles des zweiten Anteils umfaßt, während die magnetischen Mikrokügelchen in einem magnetischen Feld gehalten werden.

3. Verfahren nach Anspruch 1 oder 2, wobei die mindestens eine WBC-Population oder Unterpopulation, die von dem zweiten Anteil entfernt worden ist, ihre neutrophile Population umfaßt.

4. Verfahren nach einem der vorhergehenden Ansprüche, weiter umfassend das Abziehen von mindestens zwei WBC-Populationen oder Unterpopulationen von einem dritten Anteil der Vollblutprobe;

Analysieren des dritten Anteils nach Entfernen der roten Blutzellpopulation, um mindestens eine WBC-Populations- oder Unterpopulationscharakteristik der Vollblutprobe zu bestimmen; und
Vergleichen der drei analysierten Charakteristika, um den Prozent-Anteil von mindestens zwei WBC-Populationen oder Unterpopulationen der Vollblutprobe zu bestimmen.

5. Verfahren nach Anspruch 4, wobei die WBC-Populationen oder Unterpopulationen von dem dritten Anteil durch Zumischen von Mikrokügelchen mit einem daran gebundenen monoclonalen Antikörper, der spezifisch für die WBC-Populationen oder Unterpopulationen ist, entfernt werden, und Entfernen mindestens eines Teils des dritten Anteils, während die magnetischen Mikrokügelchen in einem magnetischen Feld gehalten werden.

6. Verfahren nach Anspruch 4 oder Anspruch 5, wobei die mindestens zwei WBC-Populationen oder Unterpopulationen, die von dem dritten Anteil abgezogen worden sind, die neutrophile und eosinophile Populationen enthalten.

7. Verfahren nach Anspruch 4 oder Anspruch 5, wobei die mindestens zwei WBC-Populationen oder Unterpopulationen, die von dem dritten Anteil abgezogen worden sind, die lymphocytische und neutrophile Populationen enthalten.

8. Verfahren nach einem der Ansprüche 4 bis 7 weiter umfassend das Abziehen von mindestens zwei WBC-Populationen oder Unterpopulationen von einem vierten Anteil der Vollblutprobe;

   Analysieren des vierten Anteils nach Entfernen der roten Blutzellpopulation, um mindestens eine WBC-Populations- oder Unterpopulationscharakteristik der Vollblutprobe zu bestimmen; und
   Vergleichen der vier analysierten Charakteristika, um den Prozent-Anteil von mindestens drei WBC-Populationen oder Unterpopulationen der Vollblutprobe zu bestimmen.

9. Verfahren nach Anspruch 8, umfassend das Vergleichen von mindestens vier analysierten WBC-Populationscharakteristika, um den Prozent-Anteil von mindestens der eosinophilen, monocytischen, lymphocytischen, basophilen und neutrophilen WBC-Populationen der Vollblutprobe zu berechnen oder zu bestimmen, um ein fünfteiliges WBC-Differential durchzuführen.

10. Verfahren nach einem der vorhergehenden Ansprüche, wobei die mindestens eine WBC-Population oder Unterpopulation, die von dem zweiten Anteil entfernt worden ist, mindestens eine WBC-Population oder Unterpopulation enthält, welche andernfalls die Analyse der mindestens einen interessierenden WBC-Population oder Unterpopulation unklar machen würde.

11. Verfahren nach Anspruch 10, weiter umfassend das Abziehen mindestens der lymphocytischen Population von dem ersten Anteil vor der Analyse, um einen Prozent-Anteil der basophilen Population der Vollblutprobe zu erhalten.

12. Verfahren nach Anspruch 10, weiter umfassend das Abziehen mindestens einer interessierenden WBC-Unterpopulation von dem ersten Anteil vor der Analyse, um einen Prozent-Anteil der Population der interessierenden WBC-Unterpopulation zu erhalten.

13. Verfahren nach einem der vorhergehenden Ansprüche, wobei jeder Analyseschritt elektronisch ist, und zwar unter Verwendung eines einzelnen elektronischen Parameters.

14. Verfahren nach einem der Ansprüche 1 bis 12, wobei jeder Analyseschritt optisch ist, und zwar unter Verwendung eines einzelnen Lichtparameters.

## Revendications

1. Méthode pour déterminer le pourcentage d'au moins une population de globules blancs (WBC) ou une population d'un sous-groupe dans un échantillon de sang entier, qui comprend :

   l'analyse de première et seconde portions de l'échantillon, après enlèvement de la population de globules rouges sans affecter de manière importante et néfaste les qualités et/ou quantités relatives des populations de WBC et/ou des populations d'un sous-groupe, pour déterminer leurs caractéristiques respectives de population de WBC ou de population de sous-groupe; et la comparaison des deux caractéristiques analysées; où la seconde portion a été traitée en épuisant sensiblement au moins une population de WBC ou une population d'un sous-groupe, par mélange de microsphères auxquelles est lié un anticorps monoclonal spécifique de la au moins une population de WBC ou population de sous-groupe; et où chaque étape d'analyse est par technique de détection ou de dispersion de la lumière de Coulter et caractérisée par l'utilisation d'un seul paramètre électronique ou de la lumière.

**2.** Méthode selon la revendication 1, où les microsphères sont magnétiques et l'épuisement consiste à retirer au moins une partie de la seconde portion alors que les microsphères magnétiques sont maintenues dans un champ magnétique.

**3.** Méthode selon la revendication 1 ou la revendication 2, où la au moins une population de WBC ou sous-groupe épuisée de la seconde portion comprend sa population de neutrophiles.

**4.** Méthode selon toute revendication précédente comprenant de plus la soustraction d'au moins deux populations de WBC ou populations de sous-groupe d'une troisième portion de l'échantillon de sang entier;

l'analyse de la troisième portion, après enlèvement de la population de globules rouges, pour déterminer au moins une caractéristique de la population de WBC ou de la population du sous-groupe de l'échantillon de sang entier; et
la comparaison des trois caractéristiques analysées pour déterminer le pourcentage d'au moins deux populations de WBC ou populations de sous-groupe de l'échantillon de sang entier.

**5.** Méthode selon la revendication 4, où les populations de WBC ou les populations de sous-groupe sont retirées de la troisième portion par mélange de microsphères auxquelles est lié un anticorps monoclonal spécifique des populations de WBC ou des populations de sous-groupe, et en retirant au moins une partie de la troisième portion alors que les microsphères magnétiques sont maintenues dans un champ magnétique.

**6.** Méthode selon la revendication 4 ou la revendication 5, où les au moins deux populations de WBC ou populations de sous-groupe soustraites de la troisième portion comprennent les populations de neutrophiles et d'éosinophiles.

**7.** Méthode selon la revendication 4 ou la revendication 5, où les au moins deux populations de WBC ou populations de sous-groupe soustraites de la troisième portion comprennent les populations de lymphocytes et de neutrophiles.

**8.** Méthode selon l'une quelconque des revendications 4 à 7 comprenant de plus la soustraction d'au moins deux populations de WBC ou populations de sous-groupe d'une quatrième portion de l'échantillon de sang entier;

l'analyse de la quatrième portion, après enlèvement de la population de globules rouges, pour déterminer au moins une caractéristique de population de WBC ou de population de sous-groupe de l'échantillon de sang entier; et
la comparaison des quatre caractéristiques analysées, pour déterminer le pourcentage d'au moins trois populations de WBC ou populations de sous-groupe de l'échantillon de sang entier.

**9.** Méthode selon la revendication 8, comprenant la comparaison d'au moins quatre caractéristiques des populations de WBC analysées, pour calculer ou déterminer le pourcentage d'au moins les populations d'éosinophiles, monocytes, lymphocytes, basophiles et neutrophiles dans WBC dans l'échantillon de sang entier, pour accomplir une différence de WBC en cinq parties.

**10.** Méthode selon toute revendication précédente, où la au moins une population de WBC ou le sous-groupe de population épuisé en la seconde portion comporte au moins une population de WBC ou un sous-groupe de la population qui obscurcirait autrement l'analyse de la au moins une population de WBC ou population de sous-groupe d'intérêt.

**11.** Méthode selon la revendication 10, comprenant de plus la soustraction d'au moins la population de lymphocytes de la première portion, avant son analyse pour obtenir un pourcentage de population de basophiles de l'échantillon de sang entier.

**12.** Méthode selon la revendication 10, comprenant de plus la soustraction d'au moins une population du sous-groupe de WBC d'intérêt de la première portion, avant son analyse pour obtenir un pourcentage de population de la population du sous-groupe de WBC d'intérêt.

**13.** Méthode selon toute revendication précédente où chaque étape d'analyse est électronique, utilisant un seul paramètre électronique.

**14.** Méthode selon l'une des revendications 1 à 12 où chaque étape d'analyse est optique, utilisant un seul paramètre

de lumière.

# FIG. 1

```
┌─────────────────┐                    ┌─────────────────┐
│   BIOLOGICAL    │──502               │    REACTANT     │──506
│     SAMPLE      │                    │                 │
└─────────────────┘                    └─────────────────┘
        │          504                         │
        │     ┌────────────────────────────────┘──508
        ↓     │                          ↓
        └─────┴──────────┬───────────────┘
                         │
                         ↓
             ┌─────────────────────┐
    ┌───▶    │     REMOVE RBC      │──510
            └─────────────────────┘
   500                   │
                         ↓
┌──────────────────────────────────────────────────────────────────────────────┐
│   ┌─518              ┌─526                    ┌─532                             │
│   ↓                  ↓                        ↓                                 │
│ ┌────────────────┐ ┌────────────────┐     ┌────────────────┐                  │
│ │  REMOVE N'S    │ │    REMOVE      │     │    REMOVE      │                  │
│ │                │ │   N'S + E'S    │     │   L'S + N'S    │                  │
│ └────────────────┘ └────────────────┘     └────────────────┘                  │
│         │    516           │      524              │     530                   │
│  514    │           520    │               528     │               534        │
│         ↓                  ↓                        │                          │
└─────────┴──────────────────┴────────────────────────────────────────────────┘
```

EP 0 467 952 B1

FIG. 2

23

FIG. 3A

FIG. 3B

FIG. 3C

FIG. 3D

FIG. 4A

FIG. 4B

FIG. 4C

FIG. 4D

FIG. 5A

FIG. 5B

FIG. 5C

FIG. 5D

FIG.6A

FIG.6B

FIG.6C

FIG.6D

FIG. 7

FIG. 8

FIG. 9

FIG. 10

FIG.11

FIG. 12A

FIG. 12B

FIG. 12C

FIG. 12D

FIG. 12E

FIG.13A

FIG.13B

FIG 13C

FIG 13D

FIG.13E

FIG. 14A

FIG. 14B

FIG. 14C

FIG. 14D

FIG. 14E

FIG.15

FIG. 16A

FIG. 16B

FIG. 16C

FIG. 16D

FIG. 16E

FIG. 16F

34

FIG.16G

FIG.17A

FIG.17B

FIG.17C

FIG.17D

FIG.17E

FIG.17F

FIG.17G

FIG. 18

FIG.19A

FIG.19B

FIG.19C

FIG 19D

FIG. 20A

FIG. 20B

FIG. 20C

FIG. 20D